(19) Europäisches Patentamt
European Patent Office
Office européen des brevets

(11) EP 4 393 508 A1

(12) EUROPEAN PATENT APPLICATION
published in accordance with Art. 153(4) EPC

(43) Date of publication:
**03.07.2024 Bulletin 2024/27**

(21) Application number: **22861085.3**

(22) Date of filing: **02.08.2022**

(51) International Patent Classification (IPC):
***A61K 45/00*** *(2006.01)* ***A61K 31/4439*** *(2006.01)*
***A61K 31/7088*** *(2006.01)* ***A61K 48/00*** *(2006.01)*
***A61P 1/16*** *(2006.01)* ***A61P 35/00*** *(2006.01)*
***A61P 43/00*** *(2006.01)* ***C12Q 1/06*** *(2006.01)*
***C12Q 1/48*** *(2006.01)* ***G01N 33/15*** *(2006.01)*
***G01N 33/50*** *(2006.01)*

(52) Cooperative Patent Classification (CPC):
**A61K 31/4439; A61K 31/7088; A61K 45/00; A61K 48/00; A61P 1/16; A61P 35/00; A61P 43/00; C12Q 1/06; C12Q 1/48; G01N 33/15; G01N 33/50**

(86) International application number:
**PCT/JP2022/029655**

(87) International publication number:
**WO 2023/026794 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.08.2021 JP 2021135496**

(71) Applicant: **OSAKA UNIVERSITY**
**Suita-shi**
**Osaka 565-0871 (JP)**

(72) Inventors:
- **KODAMA Takahiro**
**Suita-shi, Osaka 565-0871 (JP)**
- **SHIODE Yuto**
**Suita-shi, Osaka 565-0871 (JP)**
- **TAKEHARA Tetsuo**
**Suita-shi, Osaka 565-0871 (JP)**

(74) Representative: **Arnold & Siedsma**
**Bezuidenhoutseweg 57**
**2594 AC The Hague (NL)**

Remarks:
The complete document including Reference Table(s) and the Sequence Listing(s) can be downloaded from the EPO website

# (54) MATURE-HEPATOCYTE TRANSDIFFERENTIATION SUPPRESSING COMPOSITION

(57) It is an object to provide a composition that suppresses the transdifferentiation of a mature hepatocyte or a composition that can treat cholangiocarcinoma, and it is another object to provide a method of screening a substance for the suppression of the transdifferentiation of a mature hepatocyte or a substance for the treatment of cholangiocarcinoma. Provided is a composition that suppresses the transdifferentiation of a mature hepatocyte or a composition for the treatment of cholangiocarcinoma, the composition including an NF-κB inducing kinase inhibitor (NIK inhibitor). Also provided is a method of screening a substance that suppresses the transdifferentiation of a mature hepatocyte or a substance that treats cholangiocarcinoma, the method including a step of measuring the activity of an NF-κB inducing kinase. The composition of the present invention alleviates the dysregulation of a TRAF3-NIK axis involved in the transdifferentiation of a mature hepatocyte, and the development and exacerbation of cholangiocarcinoma.

EP 4 393 508 A1

FIG. 11

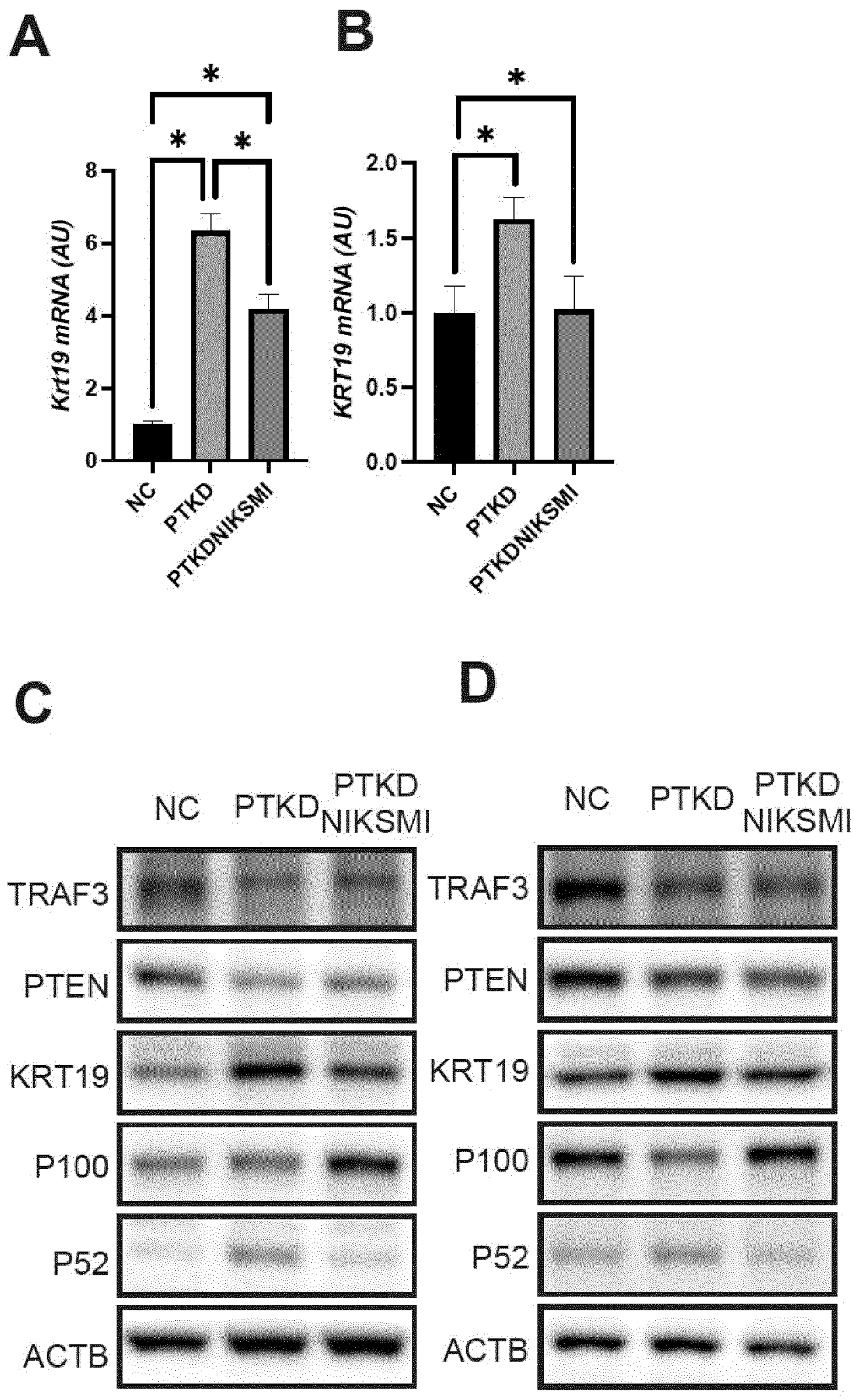
A
Krt19 mRNA (AU)
0
2
4
6
8
NC
PTKD
PTKDNIKSMI
*
*
*
B
KRT19 mRNA (AU)
0.0
0.5
1.0
1.5
2.0
NC
PTKD
PTKDNIKSMI
*
*
C
NC
PTKD
PTKD NIKSMI
TRAF3
PTEN
KRT19
P100
P52
ACTB
D
NC
PTKD
PTKD NIKSMI
TRAF3
PTEN
KRT19
P100
P52
ACTB

## Description

### Technical Field

The present invention relates to suppression of transdifferentiation of a mature hepatocyte and treatment of cholangiocarcinoma.

### Background Art

Cholangiocarcinoma accounts for 150 of primary hepatic cancers, and its number is second largest after that of hepatocellular carcinoma (Non Patent Literature 1). The cholangiocarcinoma is often found after its progression and is resistant to chemotherapy, and hence accounts for 2% of cancer-related deaths in the world (Non Patent Literature 2). The elucidation of the pathogenic mechanism of the cholangiocarcinoma may lead to its treatment, but the mechanism via which the cholangiocarcinoma occurs has many unclear points. In addition, possible candidates for a cell serving as the origin of the cholangiocarcinoma include an intrahepatic cholangiocyte, a mature hepatocyte, and a hepatic progenitor cell that are present in a liver, but no clear conclusion has been drawn. Several oncogenes have been identified from the sequence analysis of the cholangiocarcinoma (Non Patent Literature 3), but the number of therapeutic targets for which drugs can be developed is small, and hence a search for a new therapeutic target may be required.

A transposon insertional mutagenesis screening method has been reported as a method by which oncogenes are identified in various carcinomas (Non Patent Literature 4). The inventors of the present invention have searched for the oncogenes of various liver cancers on the basis of a transposon insertional mutagenesis screening method including using a liver-specific Pten-deficient mouse (Non Patent Literature 5). Further, the inventors of the present invention have performed the genomic analysis of a liver cancer having a bile duct component on the basis of the same method, and have observed inactive mutation most frequently in a Traf3 gene. Thus, the inventors have suggested that the inactivation of the Traf3 is related to the transdifferentiation of a hepatocyte and the development of intrahepatic cholangiocarcinoma (Non Patent Literature 6).

### Citation List

### Non Patent Literature

[NPL 1] Rizvi S, et al. Nat Rev Clin Oncol. 2018; 15(2): 95-111.
[NPL 2] Banales JM, et al. Nat Rev Gastroenterol Hepatol. 2020; 17(9): 557-88.
[NPL 3] Farshidfar F, et al. Cell Rep. 2017; 19(13): 2878-80.
[NPL 4] Kodama T, et al. Gastroenterology. 2016; 151(2): 324-37 e12.
[NPL 5] Kodama T, et al. Proc Natl Acad Sci U S A. 2016; 113(24): E3384-93.
[NPL 6] Shiode Yuto, et al., The 57th Annual Meeting of the Japan Society of Hepatology, WS9-10, 2021


### Summary of Invention

### Technical Problem

An object of the present invention is to elucidate the mechanism of the transdifferentiation of a mature hepatocyte to provide a composition and a method that contribute to the treatment and research of a disease associated with the transdifferentiation of a hepatocyte, the disease being typified by cholangiocarcinoma, and the development of a therapeutic drug therefor.

### Solution to Problem

The inventors of the present invention have made investigations with a view to achieving the above-mentioned object, and as a result, have found that an NF-κB inducing kinase (hereinafter also referred to as "NIK") inhibitor can suppress the transdifferentiation of a mature hepatocyte. Thus, the inventors have completed the present invention. In addition, the inventors have found that the measurement of the activity of NIK enables the screening of a substance that suppresses the transdifferentiation of a mature hepatocyte. Thus, the inventors have completed the present invention. That is, the present invention includes the following aspects.

1. A composition for suppression of transdifferentiation of a mature hepatocyte, comprising an NF-κB inducing kinase inhibitor.
2. The composition according to the above-mentioned item 1, wherein the composition suppresses transdifferentiation of the mature hepatocyte into a cholangiocyte.
3. The composition according to the above-mentioned item 2, wherein the cholangiocyte is a proliferative cholangiocyte.
4. The composition according to the above-mentioned item 1, wherein the composition is a composition for treatment of cholangiocarcinoma.
5. The composition according to the above-mentioned item 4, wherein the cholangiocarcinoma is intrahepatic cholangiocarcinoma.
6. The composition according to the above-mentioned item 4 or 5, wherein the cholangiocarcinoma is low-TRAF3 expression cholangiocarcinoma or high-NIK expression cholangiocarcinoma.
7. The composition according to any one of the above-mentioned items 1 to 3, wherein the composition is a research reagent.
8. The composition according to any one of the above-mentioned items 1 to 7, wherein the NF-κB inducing kinase inhibitor is a low-molecular weight compound that inhibits enzyme activity of an NF-κB inducing kinase.
9. The composition according to the above-mentioned item 8, wherein the low-molecular weight compound is a compound represented by the following formula (I) or a pharmaceutically acceptable salt thereof:

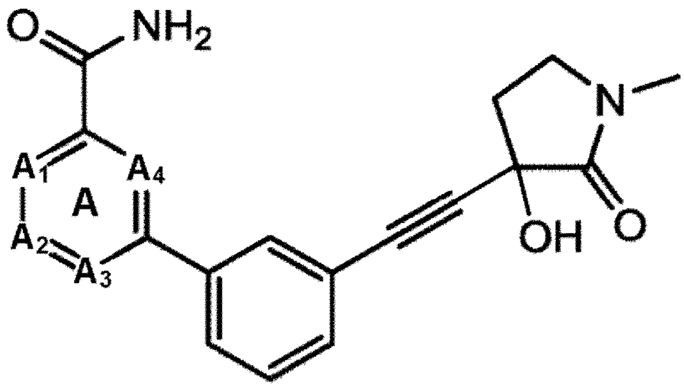

(I)

where:

a ring A is a monocycle or a fused bicycle;
$A_1$ represents N or $CR^1$;
$A_2$ represents N or $CR^2$;
$A_3$ represents N or $CR^3$;
$A_4$ represents N; and
one or two of $A_1$ to $A_4$ each represent N,

where:

$R^1$ represents H or a group that forms a ring together with $R^2$;
$R^2$ is selected from the group consisting of H, $C_1$-$C_6$ alkyl group, $C_1$-$C_6$ haloalkyl group, $C_1$-$C_6$ alkoxy group, $NR^aR^b$, heteroatom-containing 3-11 membered ring, a group that forms a ring together with $R^1$ and a group that forms a ring together with $R^3$, wherein each of $R^2$ is optionally substituted with $R^c$; and
$R^3$ is selected from the group consisting of H, halogen, and a group that forms a ring together with $R^2$,

where:

the ring that $R^1$ and $R^2$ form together is selected from the group consisting of $C_3$-$C_7$ cycloalkyl group, phenyl group and heteroatom-containing 3-11 membered ring, wherein the ring is optionally substituted with $R^d$; and
the ring that $R^2$ and $R^3$ form together is $C_3$-$C_6$ cycloalkyl group or heteroatom-containing 3-6 membered ring that is optionally substituted with $R^d$, and

where:

$R^a$ is selected from the group consisting of H and $C_1$-$C_6$ alkyl group;
$R^b$ is selected from the group consisting of H, $C_1$-$C_6$ alkoxy group, $C_3$-$C_6$ cycloalkyl group, heteroatom-containing 3-11 membered ring and $C_1$-$C_6$ alkyl group that is optionally substituted with $C_1$-$C_6$ alkoxy group;
$R^c$ is selected from the group consisting of halogen, OH, C(O) ($C_1$-$C_6$ alkyl group), heteroatom-containing 3-11 membered ring and $C_1$-$C_6$ alkyl group that is optionally substituted with $C_1$-$C_6$ alkoxy group; and
$R^d$ is selected from the group consisting of halogen, $C_1$-$C_6$ alkyl group and $C_1$-$C_6$ alkoxy group.

10. The composition according to the above-mentioned item 9, wherein $A_1$ represents CH, $A_2$ represents $COCH_3$, $A_3$ represents CH, and $A_4$ represents N.
11. The composition according to any one of the above-mentioned items 1 to 7, wherein the NF-κB inducing kinase inhibitor is a nucleic acid that inhibits translation of mRNA of an NF-κB inducing kinase.
12. A composition for treatment of cholangiocarcinoma, comprising an NF-κB inducing kinase inhibitor.
13. The composition according to the above-mentioned item 12, wherein the cholangiocarcinoma is low-TRAF3 expression cholangiocarcinoma or high-NIK expression cholangiocarcinoma.
14. A method of screening a substance that suppresses transdifferentiation of a mature hepatocyte, comprising a step of measuring activity of an NF-κB inducing kinase.
15. A method of screening a substance for treatment of cholangiocarcinoma, comprising a step of measuring activity of an NF-κB inducing kinase.
16. A method of suppressing transdifferentiation of a mature hepatocyte, comprising administering an NIK inhibitor.
17. A method of treating cholangiocarcinoma, comprising administering an NIK inhibitor.
18. A use of an NIK inhibitor for preparation of a composition for suppression of transdifferentiation of a mature hepatocyte.
19. A use of an NIK inhibitor for preparation of a composition for treatment of cholangiocarcinoma.

## Advantageous Effects of Invention

The composition of the present invention comprising the NIK inhibitor can suppress the transdifferentiation of a mature hepatocyte to treat a disease associated with the transdifferentiation of the mature hepatocyte. The composition of the present invention can suppress the occurrence of a proliferative cholangiocyte and reduce the proliferation potency of the cell to treat cholangiocarcinoma. The composition of the present invention can alleviate the dysregulation of a TRAF3-NIK axis involved in the transdifferentiation of a mature hepatocyte and the development and exacerbation of cholangiocarcinoma. A method of treating a cancer comprising controlling the transdifferentiation signal of a mature hepatocyte is a new cancer treatment method. The administration of the composition of the present invention is excellent in therapeutic effect and largely contributes to the treatment of many patients because the administration is a therapy based on the pathogenic mechanism of cholangiocarcinoma.
The composition of the present invention comprising the NIK inhibitor can be used in a research on the transdifferentiation of a mature hepatocyte.
A method of screening a drug targeting NIK involved in the transdifferentiation of a mature hepatocyte and the occurrence and proliferation of a cholangiocarcinoma cell contributes to the development of a novel therapeutic drug enabling the causal therapy of a disease associated with the transdifferentiation of the mature hepatocyte, the disease being typified by cholangiocarcinoma.

## Brief Description of Drawings

FIG. 1A shows trunk driver genes identified in the intrahepatic cholangiocarcinoma (ICC) and combined liver cancer (combined cancer of hepatocellular carcinoma and cholangiocellular carcinoma) of the liver of a liver-specific Pten-deficient transposon insertion-mutated mouse (liver-SB/Pten mouse). A transposon insertion site having a read count of 50 or more was selected, and 10 genes were identified. The figure shows that out of those genes, Traf3 is the most important mutant gene having the highest median read count. FIG. 1B is an illustration of a genetic map representing the site at which a transposon is inserted into a Traf3 gene locus. Each arrow indicates one transposon insertion.
FIG. 2 is HE-stained images and KRT19-stained images for showing the phenotypes of the livers of a HDKO mouse, a CDKO mouse, and a WT mouse 12 weeks after the administration of tamoxifen (TAM). A scale bar corresponds to 50 um. The term "HDKO" means a hepatocyte-specific Pten/Traf3 double knockout mouse (H-Traf3/PtenDKO). The term "CDKO" means a cholangiocyte-specific Pten/Traf3 double knockout mouse (C-Traf3/PtenDKO). The term "WT" means a wild-type mouse (mouse without Cre induction by TAM).

FIG. 3 is images of the livers of the HDKO mouse and the CDKO mouse 12 weeks after the administration of TAM. The term "HDKO" means a hepatocyte-specific Pten/Traf3 double knockout mouse (H-Traf3/PtenDKO). The term "CDKO" means a cholangiocyte-specific Pten/Traf3 double knockout mouse (C-Traf3/PtenDKO).

FIG. 4 is X-gal-stained and KRT19-stained images of the livers of the HDKO mouse and the WT mouse 12 weeks after the administration of TAM. A scale bar corresponds to 50 μm. ▲ indicates a boundary between a normal hepatocyte and a bile duct proliferation portion. The term "HDKO" means a hepatocyte-specific Pten/Traf3 double knockout ROSA26 Cre reporter mouse (H-Traf3/Pten-DKO/Rosa26-LacZ). The term "WT" means a wild-type mouse (mouse without Cre induction by TAM).

FIG. 5 shows the results of the examination of a process in which a hepatocyte of the liver of a HDKO (H-Traf3/PtenDKO) mouse differentiates into an intrahepatic cholangiocyte by single-cell RNA sequencing. FIG. 5A shows a t-SNE plot in which cells sharing the same transcriptome profile are grouped by a color. In FIG. 5B, the t-SNE plot is indicated by WT-derived cells (blue dots) and HDKO-derived cells (red dots). A large hepatocyte population present only in the HDKO mouse is shown by being surrounded by a black frame. FIG. 5C is a t-SNE plot for showing the WT-derived cells (blue dots). FIG. 5D is a t-SNE plot for showing the HDKO-derived cells (red dots).

FIG. 6 shows a t-SNE plot and Monocle pseudotime analysis. Dots each indicate a pseudotime. A blue dot indicates a shorter time lapse, and a red dot indicates a longer time lapse. In a gray-scale figure, the blue dots are indicated by black dots, yellow-green to green dots are indicated by dark gray dots, and orange to yellow dots are indicated by light gray dots.

FIG. 7 shows the expression level of the Krt19 gene of a liver organoid-derived cell. The liver organoid-derived cell is a cell obtained by: introducing a GFP-expressing lentivirus or a CRE-expressing lentivirus into a liver organoid generated from the liver of a Traf3/Pten-floxed mouse; and then culturing the resultant under a condition for differentiation into a hepatocyte. The term "GFP Hep" means a GFP-expressing lentivirus-introduced organoid-derived cell (WT cell). The term "Cre Hep" means a CRE-expressing lentivirus-introduced organoid-derived hepatocyte (Traf3/Pten-deficient cell). n=3 per group. *$P<0.05$.

FIG. 8 shows graphs for showing the gene expression level of a cholangiocyte marker KRT19 in a siRNA-introduced TRAF3/PTEN knockdown HepG2 cell (hereinafter also referred to as "HepG2") or primary human hepatocyte (hereinafter also referred to as "PHH"). The term "NC" means a control siRNA-introduced HepG2 or PHH. The terms "PTKD#1" and "PTKD#2" each mean a HepG2 or a PHH in which PTEN and TRAF3 are knocked down with Pten siRNA and Traf3 siRNA. FIG. 8A is a graph of the TRAF3/PTEN knockdown HepG2. FIG. 8B is a graph of the TRAF3/PTEN knockdown PHH. In each of FIG. 8A and FIG. 8B, n=4 per group. *$P<0.05$.

FIG. 9 shows the cell growth curve of the siRNA-introduced TRAF3/PTEN knockdown HepG2. The unit of an axis of abscissa is a time (hours). The term "NC" means a control siRNA-introduced HepG2. The terms "PTKD#1" and "PTKD#2" each mean a HepG2 in which PTEN and TRAF3 are knocked down with Pten siRNA and Traf3 siRNA. n=8 per group. *$P<0.05$.

FIG. 10 shows a Map3k14 (NIK) gene expression level and a western blot in a liver or a hepatocyte. The term "WT" means a wild-type mouse. The term "HDKO" means a hepatocyte-specific Pten/Traf3 double knockout mouse (H-Traf3/PtenDKO). The term "GFP Hep" means a GFP-expressing lentivirus-introduced liver organoid-derived cell (WT cell). The term "CRE Hep" means a CRE-expressing lentivirus-introduced liver organoid-derived cell (Traf3/Pten-deficient cell). The term "NC" means a control siRNA-introduced HepG2 or PHH. The terms "PTKD#1" and "PTKD#2" each mean a HepG2 or a PHH in which PTEN and TRAF3 are knocked down with Pten siRNA and Traf3 siRNA. FIG. 10A shows the Map3k14 gene expression level in the HDKO mouse 2 weeks after the administration of TAM, n=3, and *$P<0.05$. FIG. 10B shows the Map3k14 gene expression level in the HDKO mouse 12 weeks after the administration of TAM, n=4, and *$P<0.05$. FIG. 10C shows the Map3k14 gene expression levels in the GFP Hep and the CRE Hep, n=3 in each group, and *$P<0.05$. FIG. 10D shows the Map3k14 gene expression level in the TRAF3/PTEN knockdown HepG2 (left) or PHH (right), n=4 in each group, and *$P<0.05$. FIG. 10E is western blots of NIK and ACTB in the TRAF3/PTEN knockdown HepG2 or PHH.

FIG. 11 shows that an increase in expression amount of the cholangiocyte marker KRT19 in the HepG2 or the PHH by the knockdown of TRAF3 and PTEN is suppressed by NIK activity inhibition. The term "NC" means a negative control siRNA-introduced HepG2 or PHH. The term "PTKD" means a HepG2 or a PHH in which PTEN and TRAF3 are knocked down with Pten siRNA and Traf3 siRNA. The term "PTKDNIKSMI" means a PTKD whose NIK activity is inhibited by NIKSMI. FIG. 11A shows a KRT19 gene expression level in the PTKD (HepG2) to which NIKSMI is added or not added, n=4 in each group, and *$P<0.05$. FIG. 11B shows a KRT19 gene expression level in the PTKD (PHH) to which NIKSMI is added or not added, n=4 in each group, and *$P<0.05$. FIG. 11C is western blots (TRAF3, PTEN, KRT19, P100, P52, and ACTB) of the PTKD (HepG2) to which NIKSMI is added or not added. FIG. 11D is western blots (TRAF3, PTEN, KRT19, P100, P52, and ACTB) of the PTKD (PHH) to which NIKSMI is added or not added.

FIG. 12 shows that an increase in expression amount of the cholangiocyte marker KRT19 in the HepG2 by the knockdown of TRAF3 and PTEN is suppressed by NIK activity inhibition. The term "NC" means a negative control

siRNA-introduced HepG2. The term "PTKD" means a HepG2 in which PTEN and TRAF3 are knocked down with Pten siRNA and Traf3 siRNA. The term "PTKD NIK inhibitor #1" means a PTKD whose NIK activity is inhibited by an NIK inhibitor #1. The term "PTKD NIK inhibitor #2" means a PTKD whose NIK activity is inhibited by an NIK inhibitor #2. The term "PTKD NIK inhibitor #3" means a PTKD whose NIK activity is inhibited by an NIK inhibitor #3. n=3 in each group, and *P<0.05. FIG. 12A shows a KRT19 gene expression level. FIG. 12B shows a PTEN gene expression level. FIG. 12C shows a TRAF3 gene expression level. FIG. 12D is western blots (P100, P52, and ACTB) .

FIG. 13 shows that an increase in expression amount of the cholangiocyte marker KRT19 in the HepG2 or the PHH by the knockdown of TRAF3 and PTEN is suppressed by NIK knockdown. The term "NC" means a control siRNA-introduced HepG2 or PHH. The term "PTKD" means a HepG2 or a PHH in which PTEN and TRAF3 are knocked down with Pten siRNA and Traf3 siRNA. The terms "PTKDNIK#1" and "PTKDNIK#2" mean PTKDs in each of which NIK is knocked down through the introduction of NIK siRNA (NIK KD#1 and NIK KD#2, respectively). FIG. 13A shows a KRT19 gene expression level in the PTKD (HepG2) to which NIK siRNA is introduced or not introduced, n=4 in each group, and *P<0.05. FIG. 13B shows a KRT19 gene expression level in the PTKD (PHH) to which NIK siRNA is introduced or not introduced, n=4 in each group, and *P<0.05. FIG. 13C is western blots (TRAF3, PTEN, KRT19, P100, P52, NIK, and ACTB) of the PTKD (HepG2) to which NIK siRNA is introduced or not introduced. FIG. 13D is western blots (TRAF3, PTEN, KRT19, P100, P52, NIK, and ACTB) of the PTKD (PHH) to which NIK siRNA is introduced or not introduced.

FIG. 14 shows graphs for showing that an increase in proliferation rate of the HepG2 by the knockdown of TRAF3 and PTEN is blocked by NIK activity inhibition or NIK knockdown. The unit of an axis of abscissa is a time (hours). The term "NC" means a control siRNA-introduced HepG2. The term "PTKD" means a HepG2 in which PTEN and TRAF3 are knocked down with Pten siRNA and Traf3 siRNA. The term "PTKDNIKSMI" means a PTKD whose NIK activity is inhibited by the addition of NIKSMI. The terms "PTKDNIK#1" and "PTKDNIK#2" mean PTKDs in each of which NIK is knocked down through the introduction of NIK siRNA (the NIK KD#1 and the NIK KD#2, respectively). FIG. 14A shows that the increase in cell proliferation rate is blocked by the NIK activity inhibition. FIG. 14B shows that the increase in cell proliferation rate is blocked by the NIK knockdown. n=8 in each group, and *P<0.05.

FIG. 15 shows stained images of the liver of an NIKSMI-administered DKO (L-Traf3/PtenDKO) mouse and a graph for showing the bile duct marker expression level thereof. FIG. 15A shows the HE-stained images and KRT19-stained images of the livers of a DKO mouse and the NIKSMI-administered DKO mouse. A scale bar corresponds to 50 um. FIG. 15B shows the KRT19 gene expression levels of the livers of the DKO mouse and the NIKSMI-administered DKO mouse. n=5 in each group, and *P<0.05.

FIG. 16 shows HE-stained images and immunohistologically stained images of cancer tissues resected from patients with intrahepatic cholangiocarcinoma, and a graph for showing a correlation between the TRAF3 staining intensity and NIK staining intensity of each of the tissues. The upper panels of FIG. 16A shows the images of a cancer tissue having a low TRAF3 expression amount and a high NIK expression amount, and the lower panels thereof are the images of a cancer tissue having a high TRAF3 expression amount and a low NIK expression amount. A scale bar corresponds to 50 um. FIG. 16B shows a correlation between the staining intensities, and shows a negative correlation between the TRAF3 expression amount and the NIK expression amount.

FIG. 17 shows graphs for showing the disease-free survivals and overall survivals of the patients after the resection of intrahepatic cholangiocarcinoma. FIG. 17A shows a disease-free survival based on the TRAF3 staining intensity. FIG. 17B shows an overall survival based on the TRAF3 staining intensity. FIG. 17C shows a disease-free survival based on the NIK staining intensity. FIG. 17D shows an overall survival based on the NIK staining intensity.

FIG. 18 shows the western blots and cell growth curve of a human cholangiocarcinoma cell line HuCCT1 in which NIK is knocked down through the introduction of siRNA. The unit of an axis of abscissa is a time (hours). The term "NC" means a control siRNA-introduced HuCCT1. The terms "NIK KD#1" and "NIK KD#2" mean HuCCTls in each of which NIK is knocked down through the introduction of siRNA (the NIK KD#1 and the NIK KD#2, respectively). FIG. 18A is the western blots of NIK. FIG. 18B is a cell growth curve. n=8 in each group, and *P<0.05.

FIG. 19 shows the western blots and cell growth curve of a human cholangiocarcinoma cell line HuCCA1 in which NIK is knocked down through the introduction of siRNA. The unit of an axis of abscissa is a time (hours). The term "NC" means a control siRNA-introduced HuCCA1. The terms "NIK KD#1" and "NIK KD#2" mean HuCCAls in each of which NIK is knocked down through the introduction of siRNA (the NIK KD#1 and the NIK KD#2, respectively). FIG. 19A is the western blots of NIK. FIG. 19B is a cell growth curve. n=8 in each group, and *P<0.05.

FIG. 20 shows the western blots and cell growth curve of a human cholangiocarcinoma cell line KKU213 in which NIK is knocked down through the introduction of siRNA. The unit of an axis of abscissa is a time (hours). The term "NC" means a control siRNA-introduced KKU213. The terms "NIK KD#1" and "NIK KD#2" mean KKU213s in each of which NIK is knocked down through the introduction of siRNA (the NIK KD#1 and the NIK KD#2, respectively). FIG. 20A is the western blots of NIK. FIG. 20B is a cell growth curve. n=8 in each group, and *P<0.05.

FIG. 21 shows the cell growth curves of the human cholangiocarcinoma cell lines whose NIK activities are inhibited by adding 9 μM or 15 μM of NIKSMI. The unit of an axis of abscissa is a time (hours). n=8 in each group, and

*P<0.05. FIG. 21A corresponds to the HuCCT1. FIG. 21B corresponds to the HuCCA1. FIG. 21C corresponds to the KKU213.

FIG. 22 shows the cell growth curves of the human cholangiocarcinoma cell lines whose NIK activities are inhibited by adding 20 μM of any one of the NIK inhibitor #1, the NIK inhibitor #2, or the NIK inhibitor #3. The unit of an axis of abscissa is a time (hours). n=8 in each group, and *P<0.05. FIG. 22A corresponds to the HuCCT1. FIG. 22B corresponds to the HuCCA1. FIG. 22C corresponds to the KKU213.

FIG. 23 shows results obtained by orally administering NIKSMI to a NOG mouse subjected to the subcutaneous transplantation of a HuCCT1 cell. The term "NIK SMI(+)" means a mouse to which NIKSMI is orally administered. The term "NIK SMI (-)" means a vehicle-administered mouse. FIG. 23A shows a tumor size, n=7 in each group, and *P<0.05. FIG. 23B shows the number of cells positive for a cell proliferation ability marker Ki67 in a tumor tissue, n=7 in each group, and *P<0.05.

FIG. 24 shows results obtained by subcutaneously transplanting, to a NOG mouse, a HuCCT1 cell having introduced thereinto shRNA by a lentivirus. The term "NC" means a mouse to which a HuCCT1 cell having introduced thereinto negative control shRNA is transplanted. The term "NIK KD" means a mouse to which a HuCCT1 cell having introduced thereinto NIK shRNA is transplanted. FIG. 24A shows a tumor size, n=7 in each group, and *P<0.05. FIG. 24B shows the number of cells positive for the cell proliferation ability marker Ki67, n=7 in each group, and *P<0.05.

Description of Embodiments

A composition of the present invention comprises an NF-κB inducing kinase (NIK) inhibitor. NIK is one serine/threonine kinase, and is also known as Map3k14. NIK is an enzyme having an important role in a noncanonical NF-κB signaling pathway.

The NIK inhibitor serving as an active ingredient of the composition of the present invention only needs to be a substance that suppresses the function of NIK, and the inhibitor is not limited. Examples thereof include: a substance that inhibits NIK activity; a substance that inhibits the translation of the mRNA of NIK into a protein; a substance that inhibits the transcription of an NIK gene; a protein knockdown substance; an anti-NIK antibody; and an NIK-producing enzyme inhibitor.

The substance that inhibits NIK activity only needs to be a substance that suppresses the activity of NIK, and the substance is not limited. The substance is preferably a low-molecular weight compound. Examples thereof include an alkynyl alcohol derivative and a cyanoindoline derivative, and specific examples thereof include compounds described in WO 2015/025026 A1, WO 2018/037059 A1, Brightbill HD, et al. Nat Commun. 2018; 9(1): 179., and WO 2017/125530 A1. A preferred compound is, for example, a compound represented by the following formula (I):

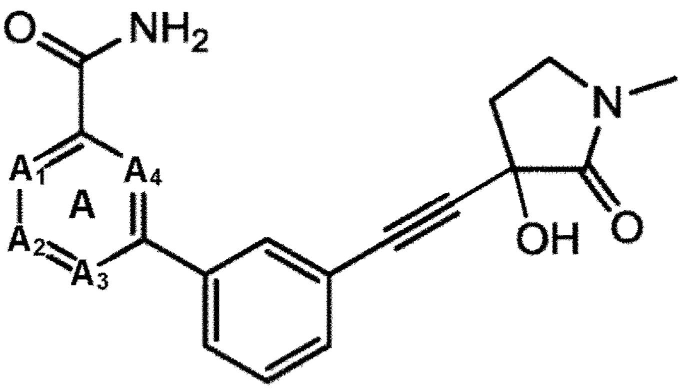

(I)

where:

a ring A is a monocycle or a fused bicycle;
$A_1$ represents N or $CR^1$;
$A_2$ represents N or $CR^2$;
$A_3$ represents N or $CR^3$;
$A_4$ represents N; and
one or two of $A_1$ to $A_4$ each represent N,

where:

$R^1$ represents H or a group that forms a ring together with $R^2$;
$R^2$ is selected from the group consisting of H, $C_1$-$C_6$ alkyl group, $C_1$-$C_6$ haloalkyl group, $C_1$-$C_6$ alkoxy group, $NR^aR^b$, heteroatom-containing 3-11 membered ring, a group that forms a ring together with $R^1$ and a group that forms a ring together with $R^3$, wherein each of $R^2$ is optionally substituted with $R^c$; and
$R^3$ is selected from the group consisting of H, halogen, and a group that forms a ring together with $R^2$,

where:

the ring that $R^1$ and $R^2$ form together is selected from the group consisting of $C_3$-$C_7$ cycloalkyl group, phenyl group and heteroatom-containing 3-11 membered ring, wherein the ring is optionally substituted with $R^d$; and
the ring that $R^2$ and $R^3$ form together is $C_3$-$C_6$ cycloalkyl group or heteroatom-containing 3-6 membered ring that is optionally substituted with $R^d$, and

where:

$R^a$ is selected from the group consisting of H and $C_1$-$C_6$ alkyl group;
$R^b$ is selected from the group consisting of H, $C_1$-$C_6$ alkoxy group, $C_3$-$C_6$ cycloalkyl group, heteroatom-containing 3-11 membered ring and $C_1$-$C_6$ alkyl group that is optionally substituted with $C_1$-$C_6$ alkoxy group;
$R^c$ is selected from the group consisting of halogen, OH, C(O) ($C_1$-$C_6$ alkyl group), heteroatom-containing 3-11 membered ring and $C_1$-$C_6$ alkyl group that is optionally substituted with $C_1$-$C_6$ alkoxy group; and
$R^d$ is selected from the group consisting of halogen, $C_1$-$C_6$ alkyl group and $C_1$-$C_6$ alkoxy group.

$A_2$ preferably represents $CR^2$. $R^2$ represents preferably a $C_1$-$C_6$ alkoxy group, more preferably a methoxy group.
A preferred compound is, for example, a compound in which $A_1$ represents CH, $A_2$ represents $COCH_3$, $A_3$ represents CH, and $A_4$ represents N. A particularly preferred compound is, for example, (R)-6-(3-((3-hydroxy-1-methyl-2-oxopyrrolidin-3-yl)ethynyl)phenyl)-4-methoxypicolinamide represented by the following formula (II) (hereinafter also referred to as a small molecule inhibitor of NIK ("NIKSMI")).

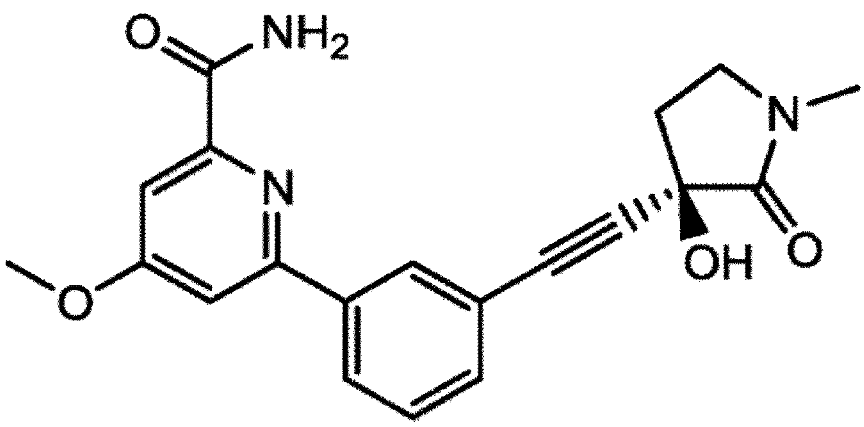

(II)

A nucleic acid that inhibits the translation of the mRNA of NIK into a protein is preferably RNA. Examples of the RNA include short hairpin RNA (shRNA), small interfering RNA (siRNA), microRNA (miRNA), antisense RNA, and a ribozyme. Preferred examples of the RNA include shRNA and siRNA. More preferred examples of the RNA are as described below.
shRNA (TRCN0000010542)

5'-CCGG-CTCAGGACTCACGTAGCATTA-CTCGAG-TAATGCTACGTGAGTCCTGAG-TTTTT-3' (SEQ ID NO: 1)

siRNA
NIK KD#1

Sense strand 5' CAAACGCAAAGAGCAAGAATT 3' (SEQ ID NO: 2)
Antisense strand 5' UUCUUGCUCUUUGCGUUUGCA 3' (SEQ ID NO: 3)

NIK KD#2

Sense strand 5' GCAAAGAGCAAGAACCAAATT 3' (SEQ ID NO: 4)
Antisense strand 5' UUUGGUUCUUGCUCUUUGCGT 3' (SEQ ID NO: 5)

Examples of the substance for protein knockdown include a proteolysis targeting chimera (PROTAC), and a specific and nongenetic IAP-dependent protein eraser (SNIPER) that are each a chimeric compound obtained by linking the ligand of NIK and the ligand of an E3 ubiquitin ligase to each other. Each of those compounds can actively add the E3 to NIK serving as a target protein to promote the decomposition of NIK.

The composition of the present invention suppresses the transdifferentiation of a mature hepatocyte. The term "mature hepatocyte" as used herein refers to a hepatocyte having an albumin-producing ability, and refers to a cell expressing a mature hepatocyte marker (e.g., Alb). It has been conceived that the mature hepatocyte does not transdifferentiate into any other cell. However, it has been recently suggested that the mature hepatocyte has such plasticity as to be transformed into any other cell (Nishikawa et al., the 109th Annual Meeting of the Japanese Society of Pathology). The mature hepatocyte in the present invention is preferably a mature hepatocyte reduced in expression of TRAF3, more preferably a mature hepatocyte reduced in expression of TRAF3 and PTEN.

The transdifferentiation of the mature hepatocyte is the conversion (transformation) of the mature hepatocyte into another kind of cell, and is, for example, transdifferentiation into a cholangiocyte, a fibroblast, an immune cell, or a sinusoidal endothelial cell. The composition suitably suppresses the transdifferentiation of the mature hepatocyte into the cholangiocyte. The composition more suitably suppresses the transdifferentiation of the mature hepatocyte into a biliary epithelial cell. The composition of the present invention still more suitably suppresses the transdifferentiation of the mature hepatocyte into a proliferative cholangiocyte. The cholangiocyte expresses cytokeratin 19 (KRT19).

A disease associated with the transdifferentiation of the mature hepatocyte is, for example, cholangiocarcinoma.

An intrahepatic cholangiocyte, a mature hepatocyte, a hepatic progenitor cell, or the like has been conceived as a candidate for a cell serving as the origin of the cholangiocarcinoma. However, the inventors of the present invention have found that the mature hepatocyte is the origin of the cholangiocarcinoma. The composition of the present invention can suppress the transdifferentiation of the mature hepatocyte to treat the cholangiocarcinoma.

In this description, the treatment of the cholangiocarcinoma includes the alleviation and mitigation of the symptom of the cholangiocarcinoma, the suppression of the exacerbation thereof, the retardation of the progression thereof, and the suppression of the recurrence thereof.

The cholangiocarcinoma is a malignant tumor (cancer) occurring in a bile duct, and is classified into "intrahepatic cholangiocarcinoma" formed in a bile duct in a liver and "extrahepatic cholangiocarcinoma" occurring in a bile duct outside the liver. The composition of the present invention can suitably treat the intrahepatic cholangiocarcinoma.

The composition of the present invention can suitably treat cholangiocarcinoma having a low TRAF3 expression amount. Alternatively, the composition can suitably treat cholangiocarcinoma having a high NIK expression amount. It has been found in the present invention that a patient suffering from the cholangiocarcinoma having a low TRAF3 expression amount has a poor prognosis, and a patient suffering from the cholangiocarcinoma having a high NIK expression amount also has a poor prognosis. The composition of the present invention can be used in the treatment of a patient who may have a poor prognosis. TRAF3 is one signaling molecule belonging to a TNF receptor-associated factor (TRAF) family.

When the amount of TRAF3 in the cholangiocarcinoma tissue or surrounding liver tissue of a patient suffering from cholangiocarcinoma is examined, and the TRAF3 amount is lower than a specified value, the administration of the composition of the present invention enables suitable treatment. When the amount of NIK in the cholangiocarcinoma tissue or surrounding liver tissue of a patient suffering from cholangiocarcinoma is examined, and the NIK amount is higher than a specified value, the administration of the composition of the present invention enables suitable treatment of the cholangiocarcinoma.

The composition of the present invention may comprise two or more kinds of active ingredients. The composition may comprise any other cancer therapeutic drug, or may comprise a drug except a cancer therapeutic drug. The composition of the present invention may be used in combination with any other cholangiocarcinoma therapeutic drug.

The composition of the present invention may comprise the NIK inhibitor and a pharmaceutically acceptable carrier.

As such carrier, carriers for pharmaceutical preparations that are typically used, for example, an excipient (e.g.:

a sugar derivative, such as mannitol or sorbitol; a starch derivative, such as corn starch or potato starch; or a cellulose derivative such as crystalline cellulose), a lubricant (e.g.: a stearic acid metal salt such as magnesium stearate; or talc), a binding agent (e.g., hydroxypropyl cellulose, hydroxypropylmethyl cellulose, or polyvinylpyrrolidone), a disintegrating agent (for example, a cellulose derivative, such as carboxymethyl cellulose or carboxymethylcellulose calcium), water, an antiseptic agent (e.g.: a p-oxybenzoic acid ester, such as methylparaben or propylparaben; or an alcohol, such as chlorobutanol or benzyl alcohol), a pH adjusting agent (e.g., an inorganic acid, such as hydrochloric acid, sulfuric acid, or phosphoric acid, an organic acid, such as acetic acid, succinic acid, fumaric acid, or malic acid, or a salt thereof), and a diluent (e.g., water for injection) may be blended alone or as a mixture thereof. The composition of the present invention encompasses a liquid formulation obtained by dissolving the active ingredient in water.

The NIK inhibitor serving as the active ingredient of the present invention may be orally administered in a dosage form, such as a tablet, a granule, a capsule, a powder, a solution formulation, a suspension formulation, or an emulsion formulation, after having been mixed with the above-mentioned carrier as required. In addition, the inhibitor may be parenterally administered in a dosage form, such as a suppository, an injection, an intravenous drip, a transdermal agent, a transmucosal agent, or an inhalant.

The active ingredient of the present invention is formulated into the above-mentioned dosage form, and is then administered to a subject in need of the ingredient, such as a human or an animal, preferably a human.

The dose, and number of times of administration, of the active ingredient of the present invention may appropriately change in accordance with conditions, such as the severity of a symptom, the age, body weight, and sex of a patient, and the kind, dosage form, and administration route of a drug. When the active ingredient is administered to a human, its dose is, for example, as follows: the ingredient is administered parenterally, for example, subcutaneously, intravenously, intraperitoneally, intramuscularly, or intrarectally in an amount of from about 0.001 mg/kg body weight to about 10 mg/kg body weight, preferably from about 0.01 mg/kg body weight to about 5 mg/kg body weight, particularly preferably from about 0.03 mg/kg body weight to about 3 mg/kg body weight per administration; and the ingredient is administered orally in an amount of from about 0.001 mg/kg body weight to about 100 mg/kg body weight, preferably from about 0.01 mg/kg body weight to about 50 mg/kg body weight, particularly preferably from about 0.1 mg/kg body weight to about 30 mg/kg body weight. In addition, the number of times of administration may be one or a plurality of times per day, for example, from once to three times, once or twice, or once per day.

The active ingredient of the present invention may be produced in accordance with a known method.

The composition of the present invention may be a research reagent. The composition of the present invention may be utilized as an agent for the suppression of the transdifferentiation of a mature hepatocyte in a research on the transdifferentiation of the mature hepatocyte.

The present application also relates to an invention of a method of screening a substance that suppresses the transdifferentiation of a mature hepatocyte, comprising a step of measuring the activity of NIK. The present application also relates to an invention of a method of screening a substance for the treatment of cholangiocarcinoma, comprising a step of measuring the activity of NIK.

The screening method of the present invention is a method of screening a substance that suppresses the transdifferentiation of a mature hepatocyte, or a method of screening a substance for the treatment of cholangiocarcinoma, out of, for example, the compounds of a compound library through the measurement of NIK-inhibiting activity.

The screening method of the present invention preferably comprises a step of bringing a test substance and NIK into contact with each other.

A method of measuring the activity of NIK is not limited, and may be a known method.

The test substance may be a compound in the compound library, and the compound library may be known or unknown. Examples of the known compound library include: a compound library (e.g., the PRESTWICK CHEMICAL LIBRARY) (this is a collection of compounds whose patent terms have expired) obtained by collecting compounds that have already obtained approval as foods (from, for example, the United States Food and Drug Administration (FDA)) or drugs (from, for example, the European Medicines Agency (EMEA)); and a compound library obtained by collecting compounds that have not obtained such approval as foods or drugs yet.

The screening method of the present invention may further comprise a step of sorting a compound on the basis of the measured activity of NIK.

The compound obtained by the screening method of the present invention may be a therapeutic drug for a disease associated with the transdifferentiation of a mature hepatocyte, or a therapeutic drug for cholangiocarcinoma. In addition, the compound obtained by the screening method of the present invention may be a research reagent.

Now, the present invention is more specifically described by way of Examples. However, the present invention is not limited thereto.

## Examples

In Test Example 1 to Test Example 8, the following materials and test methods were used.

<Human Specimen>

Formalin-fixed and paraffin-embedded human intrahepatic cholangiocarcinoma (ICC) tissues were collected from 43 patients with intrahepatic cholangiocarcinoma who had undergone surgical resection at the Department of Gastrointestinal Surgery in the Osaka University Graduate School of Medicine between 2011 and 2018. Those samples were obtained with the approval of the institutional research board of Osaka University Hospital. In addition, written informed consents were obtained from the respective patients. All experiments were performed on the basis of relevant guidelines and regulations.

<Mouse>

In each test, there were used: Alb-Cre/+; T2Onc2/+; Rosa26-lsl-SB11/+; Pten fl/fl mouse, Alb-Cre/+; Traf3 fl/fl mouse, Alb-Cre/+; Pten fl/fl; Traf3 fl/fl mouse, Alb-CreERT2/+; Pten fl/fl; Traf3 fl/fl mouse, K19-CreERT2/+; Pten fl/fl; Traf3 fl/fl mouse, Alb-CreERT2/+; Pten fl/fl; Traf3 fl/fl; ROSA26R/+ mouse, K19-CreERT2/+; Pten fl/fl; Traf3 fl/fl; ROSA26R/+ mouse, and Alb-Cre/+; Pten fl/fl; Traf3 fl/fl; Mlkl fl/fl mouse. NOG mice were used in a xenograft test. All the mice were housed in microisolators in ventilated racks in a specific pathogen-free facility, and were fed a regular diet. The Alb-Cre, K19-Cre ERT2, Pten fl/fl, and ROSA26R mice were obtained from the Jackson Laboratory. A method of generating the Alb-Cre ERT2 mice is as described in the previous report (Schuler M, et al. Genesis. 2004; 39(3): 167-172). The Traf3 fl/fl mice were obtained from Prof. Robert Brink (Garvan Institute of Medical Research). The Mlkl fl/fl mice were obtained from Prof. James Murphy (the Walter and Eliza Hall Institute of Medical Research). The T2Onc2 and Rosa26 lsl SB11 mice were obtained from the National Cancer Institute. The NOG mice were obtained from the Central Institute for Experimental Animals. All experiments were performed in conformity with relevant guidelines and regulations.

<Cell Line>

A human hepatoma cell line HepG2, and human cholangiocarcinoma cell lines HuCCT1, HuCCA1, and KKU213 were obtained from the JCRB Cell Bank. The HepG2 and KKU213 cells were cultured in a Dulbecco's modified Eagle's medium (DMEM; Nacalai Tesque, Inc., Japan) having added thereto 10% heat-inactivated fetal calf serum (FCS; Gibco, USA). The HuCCT1 cells were cultured in an RPMI medium (Nacalai Tesque, Inc., Japan) containing 10% FCS. The HuCCA1 cells were cultured in D-MEM/Ham's F-12 (containing L-glutamine and phenol red) (Wako Pure Chemical Industries, Ltd., Japan) having added thereto 10% FCS. All the cells were cultured with a $CO_2$ incubator at 37°C. The cells were detached with trypsin (Nacalai Tesque, Inc., Japan), and were passaged. Those cells were routinely examined for mycoplasma.

<Sequence, Read Processing, and Common Insertion Site Analysis>

Transposon insertion sites were mapped by using a splink HiSeq approach in the previous report[(1)]. A candidate cancer gene was identified by using a gCIS[(2)], and transposon driver analysis was performed by a method in the previous report[(1)]. [(1)]Kodama T, et al. Proc Natl Acad Sci USA. 2018; 115 (44): E10417-E10426. [(2)]Kodama T, et al. Gastroenterology. 2016; 151(2): 324-37 e12.

<Recovery of Primary Human Hepatocyte>

Human hepatocyte chimeric mice were generated in accordance with the protocol of the previous report (Hasegawa M, et al. Biochem Biophys Res Commun. 2011; 405(3): 405-410). Ganciclovir was administered to each of highly immunocompromised NOG mice (TK-NOG) each having a liver in which a herpes simplex virus type 1 thymidine kinase transgene had been expressed to induce liver injury, followed by the transplantation of human hepatocytes. As described in the previous report (Murai K, et al. Sci Rep. 2020; 10(1): 941), primary human hepatocytes were collected from the human hepatocyte chimeric mice each having a chimeric liver rate of 90% or more by using a two-step collagenase-pronase reflux method. It was recognized by FACS that the human hepatocytes accounted for 90% or more of the collected cells. Those cells were spread on a collagen-coated dish and cultured.

<Genetic Manipulation on Cell>

The transfection of siRNA or a plasmid into cells was performed with Lipofectamine RNAiMAX Transfection Reagent (Thermo Fisher Scientific, Inc., USA) or Lipofectamine 2000 (Thermo Fisher Scientific, Inc., USA), respectively, and with Opti-MEM (Thermo Fisher Scientific, Inc., USA). The transfection of the siRNA was performed as follows: the

cells were dispersed at a concentration of 100,000 cells/mL; 10 μM of the siRNA was mixed into 100 μL of the Opti-MEM at a ratio of 1 μL, and the mixture was left at rest for 5 minutes; and then 3 μL of the RNAiMAX was mixed into the mixture, and the whole was left at rest for 15 minutes, followed by administration to the supernatant of the previously dispersed cells. Further, a medium change was performed 24 hours thereafter.

<Generation of Organoid>

Mouse liver organoids were generated in accordance with the protocol of the previous report (Broutier L, et al. Nat Protoc. 2016; 11(9): 1724-1743). The generated mouse liver organoids were mixed with Matrigel, and the mixture was cultured in a culture medium [Ad-DMEM Ad-DMEM/F12 having added thereto B27 (Gibco, USA) and N2 (Gibco, USA), 1.25 μM N-acetylcysteine (Wako Pure Chemical Industries, Ltd., Japan), 10 mM nicotinamide (Merck & Co., Inc., USA), 50 ng/mL EGF (Gibco, USA), 50 ng/mL HGF (PeproTech, USA), 100 ng/mL FGF10 (PeproTech, USA), 10 μM forskolin (Wako Pure Chemical Industries, Ltd., Japan), 0.5 μM A83-01 (Merck & Co., Inc., USA), and 10% Rspol-controlled medium] at 37°C. The cultured product was passaged every 3 days at a concentration of from 1:4 to 1:8. Y-27632 (Wako Pure Chemical Industries, Ltd., Japan) was added to the initial culture after the organoid passage. A gene was introduced into each of the organoids by using a lentivirus. The organoids and the lentivirus were mixed, and the mixture was spread on a 24-well plate, and was centrifuged at 32°C and 600 g for 1 hour, followed by incubation at 37°C for 5 hours. Cells were selected with puromycin. Two weeks after the selection, the organoids were passaged and used in an experiment. The differentiation of the organoids into hepatocytes was performed with a differentiation medium [Ad-DMEM Ad-DMEM/F12 having added thereto B27 (Gibco, USA) and N2 (Gibco, USA), 1.25 μM N-acetylcysteine (Wako Pure Chemical Industries, Ltd., Japan), 50 ng/mL EGF (Gibco, USA), 100 ng/mL FGF10 (PeproTech, USA), 0.5 μM A83-01 (Merck & Co., Inc., USA), 10 μM DAPT (Merck & Co., Inc., USA), and 25 ng/mL BMP4 (Wako Pure Chemical Industries, Ltd., Japan)]. The organoids were cultured in a culture medium for 3 days, and then the medium was changed to a differentiation medium for 9 consecutive days. Further, the organoids were cultured in a differentiation medium having added thereto 3 μM dexamethasone (Merck & Co., Inc., USA) for 3 days.

<Immunoblotting>

A procedure for western blotting is as described in the previous report (Tanaka S, et al. Hepatology. 2016; 64(6): 1994-2014). The protein extracts of cells and animal tissues were prepared by using a lysis buffer having added thereto a protease inhibitor (Nacalai Tesque, Inc., Japan) and a phosphatase inhibitor (Nacalai Tesque, Inc., Japan). In western blotting for the detection of NIK, MG-132 (ChemScene, USA) was administered at a concentration of 5 μM 4 hours before protein recovery. The following antibodies were used in immunodetection: TRAF3 (1:1,000 Cell Signaling, #4729), PTEN (1:1,000 Cell Signaling, #9188), KRT19 (1:50,000 Abcam, #ab52625), p100/p52 (1:1,000 Cell Signaling, #4882), NIK (1:1,000 Cell Signaling, #4994), and β-actin (1:10,000 Sigma-Aldrich Co., LLC, #A5316). Anti-mouse IgG, HRP-linked whole Ab sheep (Cytiva, Japan), or anti-rabbit IgG, HRP-linked whole Ab donkey (Cytiva, Japan) was used as a secondary antibody, and luminescence analysis was performed with SuperSignal (trademark) West Pico PLUS Chemiluminescent Substrate (Thermo Fisher Scientific, Inc., USA).

<Immunostaining>

A histopathological slide was deparaffinized with xylene and ethanol. HE staining was performed with hematoxylin and eosin. In immunostaining, antigen activation was performed by heating in Target Retrieval Solution (Dako, USA), followed by blocking with bovine serum albumin and a reaction through use of a primary antibody at 4°C overnight. A frozen section was dried at room temperature, and was blocked with BSA (Nacalai Tesque, Inc., Japan), followed by a reaction through use of a primary antibody in VECTASTAIN ABC Rabbit IgG Kit (Vector Laboratories, Inc., USA). DAB Substrate Kit (Vector Laboratories, Inc., USA) was used in a staining reaction. The following antibodies were used in the immunostaining: KRT19 (1:500 Abcam, ab52625), TRAF3 (1:100 Abcam, ab217033), NIK (1:400 Novus Biologicals, NBP2-23603), p-RIP3K (1:100 Genentech), and KI67 (1:1,000 Cell Signaling, #12202S). In LacZ staining, a frozen section was dried at room temperature, and was then fixed with 0.20 glutaraldehyde (Nacalai Tesque, Inc., Japan) for 10 minutes. After that, the section was washed with PBS three times, and was incubated in an X-gal-containing β-galactosidase substrate (Takara Bio Inc., Japan) at room temperature overnight. After that, the cells were washed with PBS three times, and nuclei were stained with nuclear stain red (ScyTek Laboratories, Inc., USA). The number of Ki67-positive cells in a tumor was counted with ImageJ (Fiji). The immunostaining score of TRAF3 or NIK was calculated by multiplying a staining intensity (of from 0 to 3) by a stained area (%). Human ICC patients were grouped into two groups on the basis of the median immunostaining score of TRAF3 or NIK, and were evaluated for their disease-free survival (DFS) or overall survival (OS).

<RNA Isolation and qPCR>

Total RNA was extracted from a cell line and a human liver tissue with RNeasy Mini Kit (QIAGEN N.V., Nederland). Then, 1 μg of the total RNA was reverse-transcribed with ReverTra Ace qPCR RT Kit (Toyobo Co., Ltd., Tokyo) to synthesize complementary DNA. The mRNA expression of each of the following genes was analyzed through use of a TaqMan gene expression assay: human GAPDH (Hs02758991), human TRAF3 (Hs00936781), human PTEN (Hs02621230), human KRT19 (Hs00761767), human NIK (Hs01089753), mouse Gapdh (Mm09999915), mouse Traf3 (Mm00495752), mouse Pten (Mm00477208), mouse Krt19 (Mm00492980), mouse NIK (Mm00444166), and mouse Alb (Mm00802090). The gene expression amount of a human and the gene expression amount of a mouse were normalized to the expression amount of GAPDH.

<Bulk RNA Sequencing>

A library was prepared with TruSeq Stranded mRNA Library Prep Kit (Illumina, Inc., USA) in accordance with the manufacturer's instructions. Sequencing was performed with a HiSeq 3000 platform in a 75 bp single-end mode. A sequenced read was mapped to a mouse reference genome sequence (mm10) with TopHat v2.1.1. Fragments per kilobase of exon per million mapped fragments (FPKMs) were calculated with Cufflinks version 2.2.1.

<Single-cell RNA Sequencing>

Mice were subjected to portal vein puncture, and their livers were refluxed by a two-step collagenase method. Twenty-five milliliters of a preperfusion solution was injected into each of the livers at 6 mL/min. Subsequently, a perfusion solution having dissolved therein a collagenase (Merck & Co., Inc., Japan) was injected thereinto at 3 mL/min, and hepatocytes were recovered by centrifugation at 50 g for 1 minute. Next, the remaining tissue was cut into small pieces, and was stirred in a solution having dissolved therein a collagenase (Merck & Co., Inc., USA) and a hyaluronidase (Sigma-Aldrich Co. LLC, USA) at 37°C for 40 minutes, followed by centrifugation at 50 g for 1 minute. The pellet was removed, and then the supernatant was centrifuged at 350 g for 4 minutes, followed by the recovery of a NPC fraction. The hepatocytes and the NPC fraction were mixed at a ratio of 1:1, and the mixture was dissolved in a sorting buffer, followed by processing with BD Rhapsody (trademark) Cartridge Reagent Kit (Becton, Dickinson and Company, USA) in accordance with the manufacturer's instructions. A Rhapsody library was converted into a library for DNBSEQ with MGIEasy Universal Library Conversion Kit (App-A) (MGITech Co., Ltd., China). Sequencing was performed with a DNBSEQ-G400RS (MGITech Co., Ltd., China) platform in a 2×100 bp paired-end mode. Reads were obtained from 14,393 cells of a WT mouse and 10,083 cells of a DKO mouse. Those reads were analyzed with SeqGeq (Becton, Dickinson and Company, USA). Pieces of data on both the mice were merged, and then a poor-condition cell having a small read number was excluded. Thus, 10,364 cells remained. All the genes of those cells were analyzed with Seurat v3.2 by t-SNE while the parameters "% Genes," "% cells," and "Perplexity" were set to 50, 10%, and 30, respectively. The cells were divided into 15 clusters.

<Administration of AAV to mouse>

AAV plasmids pAAV2/8 and pHelper (the University of Pennsylvania) were transfected into HEK cells, and the recovered viral supernatant was concentrated with AAVpro Concentrator (Takara Bio Inc., Japan). A virus concentration was measured with AAVpro Titration Kit (Takara Bio Inc., Japan). Viruses were administered in an amount of $1\times10^{11}$ per mouse to its liver by tail vein injection.

<Cell Proliferation Assay>

Hepatocytes or cholangiocarcinoma cells were inoculated on a 96-well plate, and their cell proliferation was analyzed with IncuCyte (trademark) S1 (Sartorius AG, Germany) in accordance with the manufacturer's instructions.

<Xenograft Experiment>

To generate a xenograft tumor, $5\times10^6$ HuCCT1 cells were mixed with Matrigel at a ratio of 1:1, and the mixture was subcutaneously injected into each of NOG mice. The mice were randomly assigned to a control group or a group to be treated with NIKSMI (Genentech, Inc., USA) at the time point when their tumor sizes reached from 100 $mm^3$ to 300 $mm^3$. NIKSMI was orally administered to each of the mice twice a day at a dose of 200 mg/kg for 14 days. During the period, the diameters of the tumors were measured every 3 days, and the tumor sizes were each calculated by using the following equation.

$$\text{Tumor volume} = (\text{minor diameter})^2 \times (\text{major diameter})/2$$

Further, the HuCCT1 cells were infected with negative control shRNA or NIK shRNA by using a lentivirus, and the infected cells were selected with puromycin. A total of $5\times10^6$ cells were mixed with Matrigel at a ratio of 1:1, and the mixture was subcutaneously injected into each of the NOG mice. The measurement of the diameters of the tumors of the mice was started 3 weeks after the transplantation, and their tumor volumes were measured by the same method as that described above.

<Quantification and Statistical Analysis>

Data was represented as a mean$\pm$standard deviation. All pieces of the data were subjected to appropriate statistical tests. Comparison between two groups was performed by an unpaired two-tailed T-test for normally distributed variables, or by Mann-Whiteny's U-test for non-normally distributed variables. Comparison among three groups was performed by Tukey's method. A Kaplan-Meier method and a log-rank test were used in the analysis of a difference in OS or DFS. A $\chi$-square test was used to examine a relationship between clinical data and the immunostaining intensity of TRAF3 or NIK. A difference P of less than 0.05 was regarded as being significant. Prism v.8.4.2 for Mac [GraphPad Prism, research resource identifier (RRID): SCR_002798, (GraphPad, USA)] and JMP 13 (RRID: SCR_014242, SAS Institute, USA) were used for the analysis.

<Test Example 1: Identification of Cholangiocarcinoma Suppressor Gene Traf3 by Transposon Insertional Mutagenesis Screening>

We have previously shown that transposon insertional mutagenesis significantly accelerates hepatocarcinogenesis in a liver-specific Pten knockout (KO) mouse (Horie Y, et al. J Clin Invest. 2004; 113(12): 1774-1783). To identify a gene mutated by transposon insertion, the gene being involved in the occurrence of cholangiocarcinoma, 20 liver cancers (intrahepatic cholangiocarcinoma, or a combined cancer of intrahepatic cholangiocarcinoma and hepatocellular carcinoma) each having a cholangiocarcinoma component recognized by tubular morphology and KRT19 staining were selected from the liver of a liver-specific Pten-deficient transposon insertion-mutated mouse (liver-SB/Pten mouse). Transposon insertion sites were subjected to PCR amplification by using a splink HiSeq method (Mann KM, et al. Nat Biotechnol. 2016; 34(9): 962-972.), and their sequence was determined. 369,549 transposon insertion sites (an average of 18,477 insertion sites per tumor) were identified, and 405 important candidate cancer genes (CCGs) were identified by using a gene-centric common insertion site method (Kodama T, et al. Proc Natl Acad Sci USA. 2018; 115(44): E10417-E10426) called a gCIS method. We have previously shown that a trunk driver that operates at the initial stage of tumorigenesis can be identified by focusing on a transposon insertion site having a high sequence read (Kodama T, et al. Proc Natl Acad Sci USA. 2018; 115(44): E10417-E10426). Ten trunk drivers were identified by the analysis, and Traf3 out of the drivers was identified as the most important mutant gene having the highest median read count (FIG. 1A). Most of the transposon insertions into Traf3 were located in a coding region in an antisense orientation (FIG. 1B). Our mutagenic transposon is designed so as to inactivate a gene only when inserted in the antisense orientation. Accordingly, those pieces of data strongly suggested a possibility that transposon-mediated loss-of-function mutation of Traf3 was involved in the occurrence of intrahepatic cholangiocarcinoma.

<Test Example 2: Liver-specific Traf3/Pten Deletion induces Cholangiocyte Proliferation and promotes Development of Intrahepatic Cholangiocarcinoma>

To validate the result of transposon mutagenesis screening, a liver-specific Traf3-deficient mouse was generated by using a mouse expressing Cre recombinase under the control of an albumin gene promoter (Alb-Cre). Liver-specific Traf3 knockout mice (L-Traf3KO) were born at an expected Mendelian ratio, and grew normally. However, those mice each histologically showed the proliferation of KRT19-positive intrahepatic cholangiocytes at the age of 24 weeks. A KRT19 expression level in the liver of each of the L-Traf3KO mice was significantly higher than that in a wild-type (WT) mouse. Those pieces of data suggested that the deletion of TRAF3 in a liver induced cholangiocyte proliferation. In view of the foregoing, liver-specific Traf3/Pten-deficient mice (L-Traf3/PtenDKO) were generated. Each of those mice showed liver enlargement at the age of 6 weeks, histologically showed significant cholangiocyte overgrowth in its liver, and showed a significant increase in KRT19 expression level. Most of the L-Traf3/Pten-DKO mice died at an age as early as from about 8 weeks to about 12 weeks after their birth. All of the long-living L-Traf3/Pten-DKO mice each had a plurality of tumors in its liver at the age of 24 weeks. Meanwhile, none of the L-Traf3KO mice, the liver-specific Pten knockout (L-PtenKO) mice, and the WT mice showed those phenotypes. Most of the formed tumors were diagnosed as intrahepatic carcinoma by two pathologists because of their KRT19 positivity and abnormalities in a bile duct structure.

Those pieces of data suggested that TRAF3 inactivation induced the proliferation of cholangiocytes involved in the development of intrahepatic cholangiocarcinoma.

<Test Example 3: Intrahepatic Cholangiocarcinoma develops from Traf3/Pten-deficient Hepatocyte>

To analyze the role of TRAF3 in the development of cholangiocarcinoma in a liver, the Traf3 and Pten of a hepatocyte or a cholangiocyte in the adult liver of each of mice (Alb-CreERT2 or K19-CreERT2) each expressing tamoxifen (TAM)-inducible Cre recombinase under the control of an albumin or keratin 19 promoter were specifically knocked out. TAM (500 mg/kg) or a vehicle was intraperitoneally administered to each of those mice (at the age of 6 weeks). A vehicle-administered mouse was used as a control WT mouse. A hepatocyte-specific Traf3/Pten double knockout mouse (H-Traf3/Pten-DKO, HDKO) showed liver enlargement and intrahepatic cholangiocyte overgrowth involving an increase in KRT19 expression level 12 weeks after the TAM administration. Meanwhile, a cholangiocyte-specific Traf3/Pten double knockout mouse (C-Traf3/Pten-DKO, CKDO) did not show any of those characteristics even 24 weeks after the TAM administration at the age of 6 weeks (FIG. 2). An H-Traf3/Pten-DKO mouse developed a plurality of intrahepatic cholangiocarcinomas 12 weeks after the TAM administration (FIG. 3). Hepatocyte-specific Traf3/Pten deletion recapitulated the phenotype of a liver-specific Traf3/Pten-deficient mouse (L-Traf3/PtenDKO) .

We have investigated the origin of intrahepatic cholangiocarcinoma cells in those mice, and have found that the Traf3/Pten expression levels of those tumors are significantly lower than those of wild-type liver tissues. The foregoing suggests a possibility that intrahepatic cholangiocarcinoma originates from a Traf3/Pten-deficient hepatocyte of the H-Traf3/Pten-DKO mouse. In view of the foregoing, lineage analysis was performed with a ROSA26 Cre reporter mouse (Rosa26-LacZ) (Soriano P. Nat Genet. 1999; 21(1): 70-71) expressing β-galactosidase (LacZ) in a Cre-expressing tissue. To this end, an H-Traf3/Pten-DKO/Rosa26-LacZ mouse or a C-Traf3/Pten-DKO/Rosa26-LacZ mouse was generated by crossing the Rosa26-LacZ mouse with the H-Traf3/Pten-DKO mouse or the C-Traf3/Pten-DKO mouse, respectively.

12 Weeks after the administration of TAM to each of those mice, the mouse was evaluated for its positivity for LacZ staining. In the C-Traf3/Pten-DKO/Rosa26-LacZ mouse, a cholangiocyte was LacZ-positive, but other cell types including a hepatocyte were negative. Thus, it was recognized that a K19-CreERT2 mouse expressed Cre recombinase in a cholangiocyte through the TAM administration. In contrast, in the H-Traf3/Pten-DKO/Rosa26-LacZ mouse, a hepatocyte was positive for LacZ staining, and a normal cholangiocyte was negative. Thus, it was recognized that an Alb-CreERT2 mouse expressed hepatocyte-specific Cre recombinase through the TAM administration. What is important is the fact that in the H-Traf3/Pten-DKO/Rosa26-LacZ mouse, a KRT19-positive proliferative cholangiocyte and intrahepatic cholangiocarcinoma were positive for LacZ staining (FIG. 4). Thus, it was strongly suggested that a proliferative cholangiocyte and intrahepatic cholangiocarcinoma originated from a Traf3/Pten-deficient hepatocyte.

To recognize that an intrahepatic cholangiocyte originated from the Traf3/Pten-deficient hepatocyte, Traf3 and Pten were selectively deleted from an adult hepatocyte by any other method. A Cre recombinase-expressing vector under the control of a thyroxine-binding globulin (TBG) promoter was delivered into each of the mature hepatocytes of Traf3/Pten-floxed mice by hydrodynamic tail vein injection or an adenovirus-associated vector (AAV8) (Yanger K, et al. Cell Stem Cell. 2014; 15(3): 340-349). In each of those mice, intrahepatic cholangiocarcinoma exacerbated at a high penetrance 16 weeks after the Cre delivery, and a Traf3/Pten expression level in a tumor tissue significantly reduced as compared to the expression level of a surrounding liver tissue.

The foregoing results suggest that intrahepatic cholangiocarcinoma originates from the Traf3/Pten-deficient hepatocyte.

<Test Example 4: Single-cell RNA Sequencing reveals Transdifferentiation of Traf3/Pten-deficient Hepatocyte into Cholangiocyte>

To further investigate a process in which a Traf3/Pten-deficient hepatocyte progressed to an intrahepatic cholangiocyte, the single-cell RNA sequencing of liver cells obtained from an H-Traf3/Pten-DKO mouse and a control WT littermate was performed 12 weeks after the administration of TAM. The cell type of each cluster in t-SNE clustering was annotated on the basis of the expression of a previously reported cell type marker. The t-SNE clustering identified various liver cell types common to both the mice, which included liver cells (clusters 3, 6, and 15), an endothelial cell (cluster 8), and various immune cells (clusters 10 to 14), and large hepatocyte populations (clusters 1, 2, 4, 5, 7, and 9) present only in the H-Traf3/Pten-DKO mouse (FIG. 5).

Those clusters unique to the H-Traf3/Pten-DKO mouse each expressed not only liver cell markers (Alb, Apoa1, and Ass1) but also cholangiocyte markers (Spp1, Sox9, and Onecut1). Further, pseudotime analysis was performed by a Monocle algorithm. As a result, it was suggested that a hepatocyte co-expressing those liver cell markers and cholangiocyte markers was transdifferentiated from a normal hepatocyte (FIG. 6).

<Test Example 5: TRAF3/PTEN Inhibition promotes Transdifferentiation of Hepatocytes into Cholangiocytes in Mouse Liver Organoid, Human Hepatocyte, and Human Hepatoma Cell Line>

The in vitro recapitulation of the in vivo phenotype of hepatocyte transdifferentiation by Traf3/Pten deletion in a hepatocyte lineage cell was aimed. To this end, first, an organoid was generated from the liver of a Traf3/Pten-floxed mouse in accordance with a method in the previous report (Broutier L, et al. Nat Protoc. 2016; 11(9): 1724-1743). A lentivirus vector expressing GFP or Cre was introduced into the liver organoid to generate a WT or a Traf3/Pten-deficient liver organoid. Each of those organoids was cultured under a condition for differentiation into a hepatocyte, and was then evaluated for the expression level of a cholangiocyte marker. The liver organoid-derived Traf3/Pten-deficient cell showed a KRT19 expression level significantly higher than that of the liver organoid-derived cell (WT) (FIG. 7).

The effect of TRAF3/PTEN inhibition on the expression of a bile duct marker in each of a primary human hepatocyte (hereinafter also referred to as "PHH") and a human hepatoma cell line HepG2 cell (hereinafter also referred to as "HepG2") was evaluated. The PHH and the HepG2 are each a mature hepatocyte having an albumin-producing ability. The TRAF3 and PTEN of each of the HepG2 and the PHH were knocked down with siRNA. The term "PDKD#1" means a HepG2 or a PHH having introduced thereinto siRNA Traf3#2 and Pten#1 (hereinafter also referred to as "PTKD#1HepG2" and "PTKD#1PHH" respectively). The term "PTKD#2" means a HepG2 or a PHH having introduced thereinto the siRNA Traf3#2 and Pten#3 (hereinafter also referred to as "PTKD#2HepG2" and "PTKD#2PHH" respectively). All the siRNAs were obtained from Thermo Fisher Scientific, Inc. The sequence of each of the siRNAs is described below.

Traf3#2

Sense strand 5' GGAUAAUUGCUGCAAGAGATT 3' (SEQ ID NO: 6)
Antisense strand 5' UCUCUUGCAGCAAUUAUCCTT 3' (SEQ ID NO: 7) Pten#1
Sense strand 5' GCAUACGAUUUUAAGCGGATT 3' (SEQ ID NO: 8)
Antisense strand 5' UCCGCUUAAAAUCGUAUGCAG 3' (SEQ ID NO: 9)

Pten#3

Sense strand 5' GGUUUUCGAGUCCUAAUUATT 3' (SEQ ID NO: 10)
Antisense strand 5' UAAUUAGGACUCGAAAACCTT 3' (SEQ ID NO: 11)

The TRAF3/PTEN inhibition in each of those cells significantly increased KRT19 expression (FIG. 8). Further, the TRAF3/PTEN inhibition significantly increased the proliferation rate of the HepG2 cell (FIG. 9). Those pieces of data suggested that TRAF3/PTEN inactivation served as a driving force for the transdifferentiation of a hepatocyte into a proliferative cholangiocyte.

<Test Example 6: Traf3/Pten Inactivation induces Transdifferentiation of Hepatocyte into Proliferative Cholangiocyte via NIK Activation>

An attempt was made to identify a molecular mechanism included in the transdifferentiation of a hepatocyte into a cholangiocyte. Liver tissues 2 weeks after TAM administration were collected from an H-Traf3/Pten-DKO mouse and a WT littermate. A time point 2 weeks after the TAM administration is the time point at which no clear overgrowth of a cholangiocyte in a liver is present, and a significant reduction in Traf3/Pten level is observed. We have examined the activity of a signaling pathway known to be involved in bile duct proliferation, such as a Hippo signaling pathway, a JNK pathway, or a Hedgehog signaling pathway. However, none of the pathways was dysregulated in the liver of the H-Traf3/Pten-DKO mouse. However, we have found the phosphorylation of RIPK3 (Seehawer M, et al. Nature. 2018; 562(7725): 69-75) serving as an important mediator for necroptosis that has recently been shown to be involved in the development of hepatocyte-derived cholangiocarcinoma. In addition, MLKL serving as an executer of necroptosis in the liver of an L-Traf3/Pten-DKO mouse was genetically deleted. However, the genetic deletion did not change the phenotype of the liver.

In view of the foregoing, we have performed the RNA sequence analysis of those liver tissues, and have investigated a gene whose expression amount was increased in a DKO liver as compared to a WT liver. Thus, 197 genes were identified. Of those, Map3k14 (Parvatiyar K, et al. Nat Commun. 2018; 9(1) : 2770), which was known as NF-κB inducing kinase (NIK) and served as a downstream molecule of TRAF3 regulating noncanonical NF-κB signaling, was selected for further analysis. We have recognized that the expression level of Map3k14 (NIK) was significantly upregulated in the liver of the H-Traf3/Pten-DKO mouse 12 weeks after the administration of TAM (FIG. 10A and FIG. 10B). It was recognized that the expression level of Map3k14 (NIK) was also significantly upregulated in each of the

mouse liver organoid-derived cell, the human hepatoma cell line (HepG2), and the primary human hepatocyte (PHH) obtained in Test Example 5 described above in each of which TRAF3 and PTEN were knocked down (FIG. 10C to FIG. 10E).

In view of the foregoing, it was examined whether or not the inhibition of the kinase activity of NIK with an NIK inhibitor (NIKSMI) represented by the following formula (II) changed an increase in expression of the bile duct marker KRT19 and an increase in cell proliferation rate in each of the human hepatoma cell line cell (HepG2) and the primary human hepatocyte (PHH) in each of which TRAF3 and PTEN were knocked down, the increases having been observed in Test Example 5 described above.

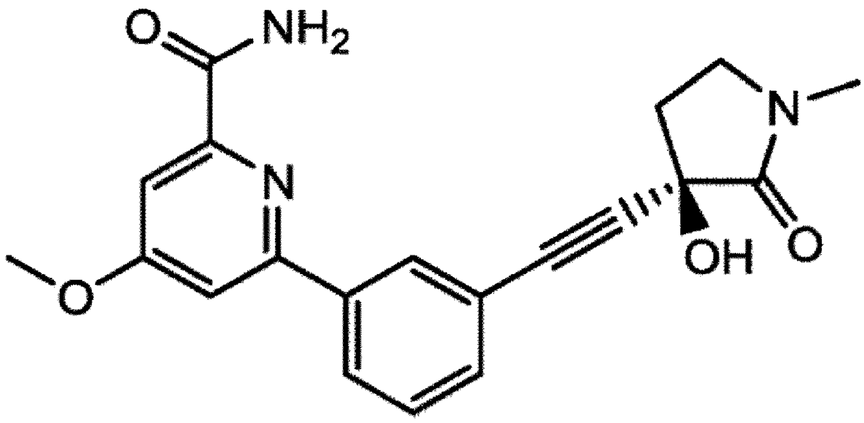

**(II)**

Specifically, 24 hours after the start of the culture of each of the PTKD#1HepG2 and the PTKD#1PHH obtained in Test Example 5, NIKSMI described above was added at a concentration of 9 μM. 48 Hours after that, the cells were recovered, and the mRNA of KRT19 was quantified, followed by immunoblotting. The results are shown in FIG. 11. Further, cell growth curves are shown in FIG. 14A.

In each of the HepG2 and the PHH, the expression of P52 and the expression of KRT19 were increased by the knockdown of TRAF3 and PTEN, but the increases were significantly suppressed by NIKSMI (FIG. 11). Further, NIKSMI blocked an increase in proliferation rate of the HepG2 cell by the knockdown of TRAF3 and PTEN (FIG. 14A).

Low-molecular weight compounds NIK inhibitor #1, NIK inhibitor #2, and NIK inhibitor #3 each having an NIK-inhibiting action were obtained, and each of the compounds was examined for a suppressing action on an increase in expression of KRT19 as in NIKSMI described above.

Specifically, 24 hours after the start of the culture of the PTKD#1HepG2 obtained in Test Example 5, the NIK inhibitor #1 was added at a concentration of 20 μM. 48 Hours after that, the cells were recovered, and the mRNA of KRT19 was quantified, followed by immunoblotting. For each of the NIK inhibitor #2 and the NIK inhibitor #3, similarly, the mRNA of KRT19 was quantified, and immunoblotting was performed. In addition, a reduction in expression of each of PTEN and TRAF3 was observed, and the NIK-inhibiting action of each of the NIK inhibitor #1, the NIK inhibitor #2, and the NIK inhibitor #3 was recognized by a suppressing action on an increase in P52. The results are shown in FIG. 12.

In the HepG2, the expression of P52 and the expression of KRT19 were increased by the knockdown of TRAF3 and PTEN, but the NIK inhibitor #1, the NIK inhibitor #2, and the NIK inhibitor #3 each significantly suppressed the increases (FIG. 12).

Next, it was examined whether or not the inhibition of expression of NIK with siRNA of NIK (NIK KD#1 and NIK KD#2) changed an increase in expression of the bile duct marker KRT19 and an increase in cell proliferation rate in each of the human hepatoma cell line (HepG2) and the primary human hepatocyte (PHH) in each of which TRAF3 and PTEN were knocked down, the increases having been observed in Test Example 5 described above. The NIK KD#1 and the NIK KD#2 were obtained from Thermo Fisher Scientific, Inc. The base sequences of the siRNAs are described below.

NIK KD#1

Sense strand 5' CAAACGCAAAGAGCAAGAATT 3' (SEQ ID NO: 2)
Antisense strand 5' UUCUUGCUCUUUGCGUUUGCA 3' (SEQ ID NO: 3) NIK KD#2
Sense strand 5' GCAAAGAGCAAGAACCAAATT 3' (SEQ ID NO: 4)
Antisense strand 5' UUUGGUUCUUGCUCUUUGCGT 3' (SEQ ID NO: 5)

Specifically, the NIK KD#1 or the NIK KD#2 was transfected into the PTKD#1HepG2 and the PTKD#1PHH obtained in Test Example 5 to provide a PTKDNIK#1 and a PTKDNIK#2, respectively. 72 Hours after the start of the

culture of each of the cells, the cell was recovered, and the mRNA of KRT19 was quantified, followed by immunoblotting. The results are shown in FIG. 13. In addition, cell growth curves are shown in FIG. 14B.

In each of the HepG2 and the PHH, the expression of P52 and the expression of KRT19 were increased by the knockdown of TRAF3 and PTEN, but the increases were significantly suppressed by the NIK KD#1 or the NIK KD#2 (FIG. 13). Further, the NIK KD#1 and the NIK KD#2 each blocked an increase in proliferation rate of the HepG2 cell by the knockdown of TRAF3 and PTEN (FIG. 14B).

In addition, we have started to orally administer NIKSMI represented by the formula (II) to an L-Traf3/PtenDKO mouse twice a day at a dose of 200 mg/kg for 14 days at the age of 4 weeks, have extracted its liver at the age of 6 weeks, and have performed the HE staining and KRT19 immunostaining of the liver. NIK inhibition significantly suppressed the overgrowth of a cholangiocyte (FIG. 15A). The expression level of the bile duct marker KRT19 in the liver of the L-Traf3/PtenDKO mouse was significantly suppressed by the administration of NIKSMI (FIG. 15B) .

Those pieces of data showed that the inactivation of TRAF3 and PTEN induced the transdifferentiation of a hepatocyte into a proliferative cholangiocyte via the activation of NIK.

<Test Example 7: Dysregulation of TRAF3-NIK Axis is associated with Advanced Stage and Poor Prognosis of Human Intrahepatic Cholangiocarcinoma>

Clinical relevance between TRAF3 and NIK was evaluated in each of 46 patients from whom intrahepatic cholangiocarcinoma had been surgically resected. First, the immunohistological staining of TRAF3 and NIK in an intrahepatic cholangiocarcinoma tissue was performed (FIG. 16A), and their expression levels were quantified. There was a negative correlation between those expression levels in a tumor tissue (FIG. 16B), and the correlation suggested that TRAF3 negatively controlled NIK in a human intrahepatic cholangiocarcinoma tissue. Next, the relevance of the expression level of TRAF3 or NIK with a clinicopathological factor or a prognosis was evaluated. A low TRAF3 expression level or a high NIK expression level was significantly associated with an advanced stage of a tumor. What is important is the fact that after the surgical resection, patients each having a low TRAF3 level or a high NIK level showed a disease-free survival (DFS) and an overall survival (OS) significantly lower than those of patients each having a high TRAF3 level or a low NIK level (FIG. 17). Those results suggested that human intrahepatic cholangiocarcinoma having the dysregulation of a TRAF3-NIK axis had a higher malignant potential.

<Test Example 8: Potential of New Treatment of Cholangiocarcinoma by NIK Inhibition>

The potential of new intrahepatic cholangiocarcinoma treatment targeting the dysregulation of a TRAF3-NIK axis was evaluated. To this end, first, the evaluation was performed with a human cholangiocarcinoma cell line in vitro.

Two kinds of NIK siRNA (the NIK KD#1 and the NIK KD#2) were introduced into each of human cholangiocarcinoma cell lines HuCCT1, HuCCA1, and KKU213 to knock down MAP3K14 (NIK). As a result, the proliferation of the respective cholangiocarcinoma cells was significantly suppressed (FIG. 18 to FIG. 20).

In addition, NIKSMI represented by the formula (II) was added at a concentration of 9 μM or 15 μM to the cell culture medium of each of the human cholangiocarcinoma cell lines HuCCT1, HuCCA1, and KKU213 at the beginning of its culture. As a result, NIKSMI significantly suppressed the proliferation of those cells (FIG. 21). Further, the NIK inhibitor #1, the NIK inhibitor #2, and the NIK inhibitor #3 were each examined for a suppressing action on the proliferation of those human cholangiocarcinoma cells. The NIK inhibitor #1, the NIK inhibitor #2, or the NIK inhibitor #3 was added at a concentration of 20 μM at the beginning of the culture. As a result, each of the NIK inhibitors significantly suppressed the proliferation of those cells (FIG. 22).

The above-mentioned cell proliferation analysis and the production of the cell growth curves were performed with IncuCyte (trademark).

Next, the effect of NIKSMI administration was investigated in vivo.

HuCCT1 cells were subcutaneously transplanted into a NOG mouse. On the 28th day after the transplantation and thereafter, NIKSMI represented by the formula (II) was orally administered to the mouse twice a day at a dose of 200 mg/kg for 14 days. During the period, the diameters of tumors were measured every 3 days, and tumor sizes were calculated. NIKSMI significantly suppressed the growth of xenografts derived from the HuCCT1 cells (FIG. 23A). The number of cells positive for a cell proliferation ability marker Ki67 was counted with ImageJ. As a result, the number of the Ki67-positive cells in the NIKSMI-administered tumors was significantly lower than that in vehicle-administered tumors (FIG. 23B).

The effect of NIK shRNA administration was investigated in vivo. HuCCT1 cells each having introduced thereinto NIK shRNA by a lentivirus or HuCCT1 cells each having introduced thereinto negative control shRNA were subcutaneously transplanted into a NOG mouse. On the 21st day after the transplantation and thereafter, the diameters of tumors were measured every 3 days, and tumor sizes were calculated. NIK shRNA significantly suppressed the growth of xenografts derived from the HuCCT1 cells (FIG. 24). The number of cells positive for the cell proliferation ability marker

Ki67 was counted with ImageJ. As a result, the number of the Ki67-positive cells in the NIK shRNA-introduced tumors was significantly lower than that in the negative control shRNA-introduced tumors (FIG. 24B). The sequence of the used shRNA is described below. 5'-CCGG-CTCAGGACTCACGTAGCATTA-CTCGAG-TAATGCTACGTGAGTCCTGAG-TTTTT-3' (SEQ ID NO: 1)

Those results suggested that the NIK inhibition was a new therapeutic strategy for cholangiocarcinoma.

Industrial Applicability

The present invention contributes to the prevention, treatment, diagnosis, and research of a disease associated with the transdifferentiation of a mature hepatocyte and cholangiocarcinoma.

## Claims

1. A composition for suppression of transdifferentiation of a mature hepatocyte, comprising an NF-κB inducing kinase inhibitor.

2. The composition according to claim 1, wherein the composition is a composition for suppression of transdifferentiation of the mature hepatocyte into a cholangiocyte.

3. The composition according to claim 2, wherein the cholangiocyte is a proliferative cholangiocyte.

4. The composition according to claim 1, wherein the composition is a composition for treatment of cholangiocarcinoma.

5. The composition according to claim 4, wherein the cholangiocarcinoma is intrahepatic cholangiocarcinoma.

6. The composition according to claim 4 or 5, wherein the cholangiocarcinoma is low-TRAF3 expression cholangiocarcinoma or high-NIK expression cholangiocarcinoma.

7. The composition according to any one of claims 1 to 3, wherein the composition is a research reagent.

8. The composition according to any one of claims 1 to 7, wherein the NF-κB inducing kinase inhibitor is a low-molecular weight compound that inhibits enzyme activity of an NF-κB inducing kinase.

9. The composition according to claim 8, wherein the low-molecular weight compound is a compound represented by the following formula (I) or a pharmaceutically acceptable salt thereof:

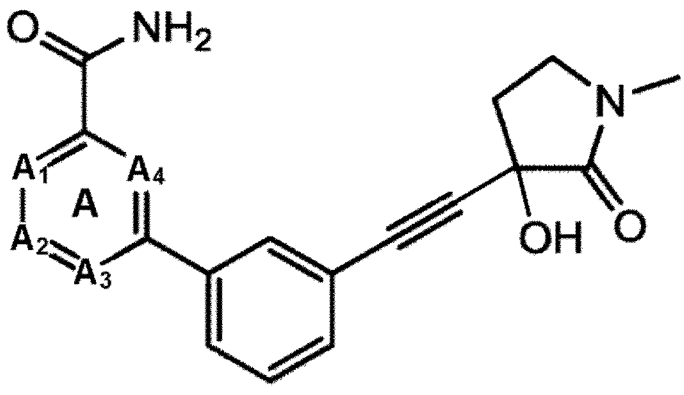

(I)

where:

a ring A is a monocycle or a fused bicycle;
$A_1$ represents N or $CR^1$;
$A_2$ represents N or $CR^2$;
$A_3$ represents N or $CR^3$;
$A_4$ represents N; and

one or two of $A_1$ to $A_4$ each represent N,

where:

$R^1$ represents H or a group that forms a ring together with $R^2$;
$R^2$ is selected from the group consisting of H, $C_1$-$C_6$ alkyl group, $C_1$-$C_6$ haloalkyl group, $C_1$-$C_6$ alkoxy group, $NR^aR^b$, heteroatom-containing 3-11 membered ring, a group that forms a ring together with $R^1$ and a group that forms a ring together with $R^3$, wherein each of $R^2$ is optionally substituted with $R^c$; and
$R^3$ is selected from the group consisting of H, halogen, and a group that forms a ring together with $R^2$,

where:

the ring that $R^1$ and $R^2$ form together is selected from the group consisting of $C_3$-$C_7$ cycloalkyl group, phenyl group and heteroatom-containing 3-11 membered ring, wherein the ring is optionally substituted with $R^d$; and the ring that $R^2$ and $R^3$ form together is $C_3$-$C_6$ cycloalkyl group or heteroatom-containing 3-6 membered ring that is optionally substituted with $R^d$, and

where:

$R^a$ is selected from the group consisting of H and $C_1$-$C_6$ alkyl group;
$R^b$ is selected from the group consisting of H, $C_1$-$C_6$ alkoxy group, $C_3$-$C_6$ cycloalkyl group, heteroatom-containing 3-11 membered ring and $C_1$-$C_6$ alkyl group that is optionally substituted with $C_1$-$C_6$ alkoxy group;
$R^c$ is selected from the group consisting of halogen, OH, C(O) ($C_1$-$C_6$ alkyl group), heteroatom-containing 3-11 membered ring and $C_1$-$C_6$ alkyl group that is optionally substituted with $C_1$-$C_6$ alkoxy group; and
$R^d$ is selected from the group consisting of halogen, $C_1$-$C_6$ alkyl group and $C_1$-$C_6$ alkoxy group.

**10.** The composition according to claim 9, wherein $A_1$ represents CH, $A_2$ represents $COCH_3$, $A_3$ represents CH, and $A_4$ represents N.

**11.** The composition according to any one of claims 1 to 7, wherein the NF-κB inducing kinase inhibitor is a nucleic acid that inhibits translation of mRNA of an NF-κB inducing kinase.

**12.** A composition for treatment of cholangiocarcinoma, comprising an NF-κB inducing kinase inhibitor.

**13.** The composition according to claim 12, wherein the cholangiocarcinoma is low-TRAF3 expression cholangiocarcinoma or high-NIK expression cholangiocarcinoma.

**14.** A method of screening a substance that suppresses transdifferentiation of a mature hepatocyte, comprising a step of measuring activity of an NF-κB inducing kinase.

**15.** A method of screening a substance for treatment of cholangiocarcinoma, comprising a step of measuring activity of an NF-κB inducing kinase.

FIG. 1

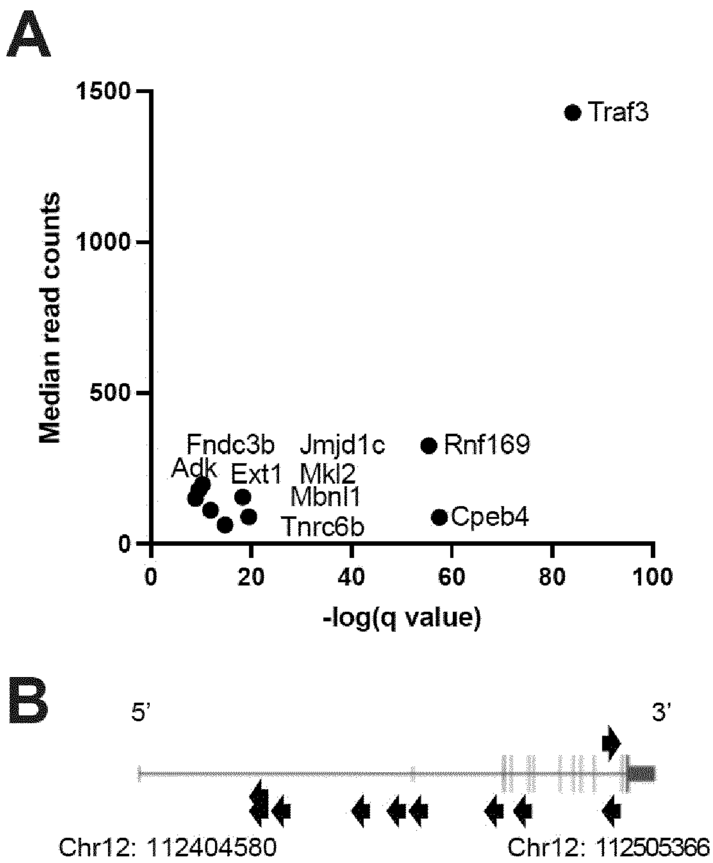
A
Median read counts
1500
1000
500
0
-log(q value)
0
20
40
60
80
100
Traf3
Rnf169
Cpeb4
Fndc3b
Jmjd1c
Adk
Ext1
Mkl2
Mbnl1
Tnrc6b
B
5'
3'
Chr12: 112404580
Chr12: 112505366

FIG. 2

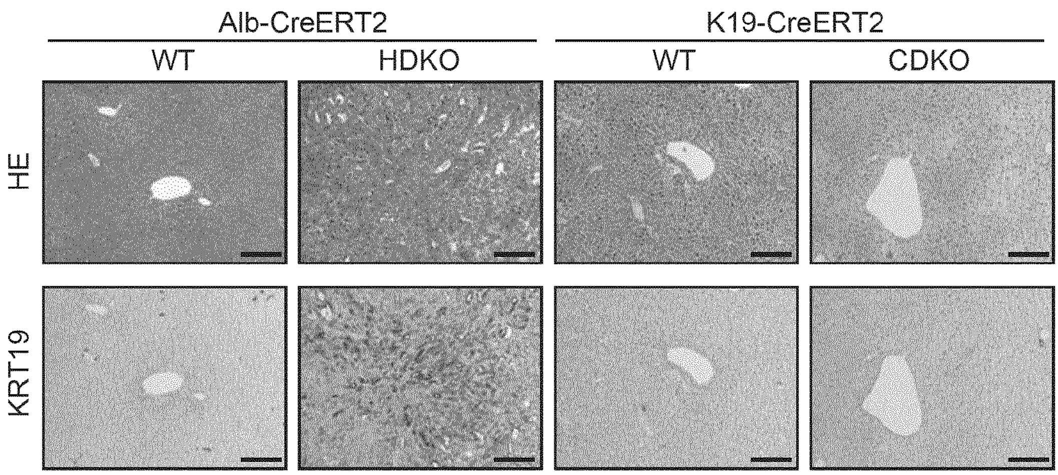
Alb-CreERT2
K19-CreERT2
WT
HDKO
WT
CDKO
HE
KRT19

FIG. 3

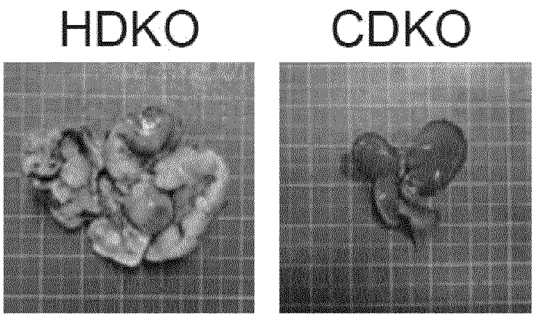


FIG. 4

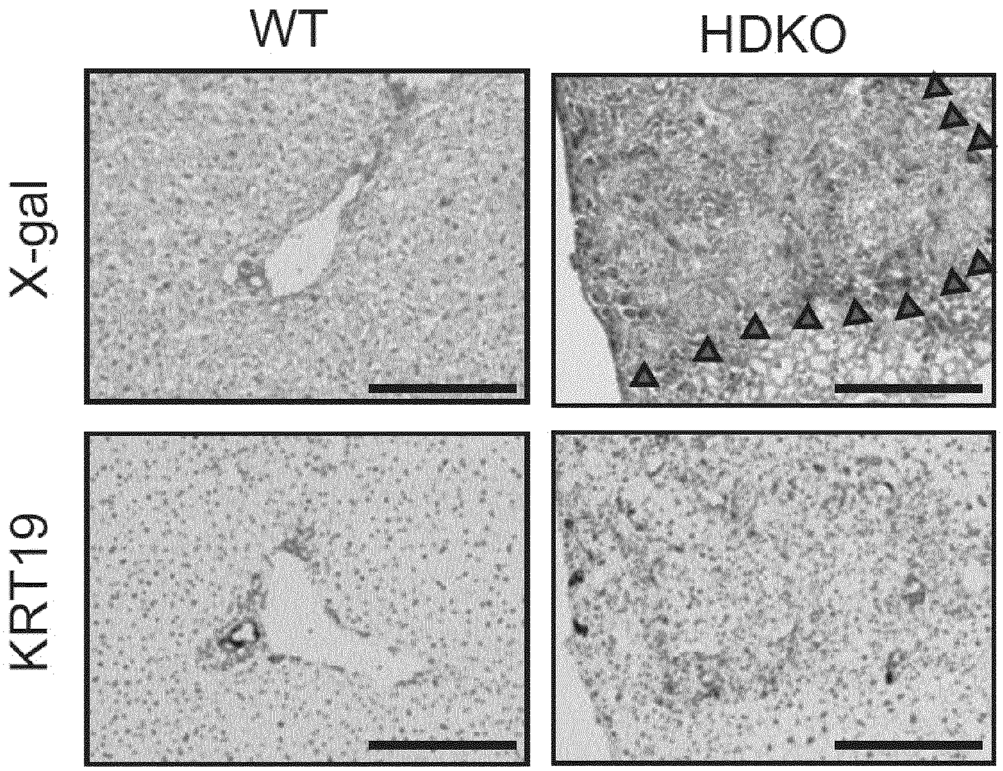

FIG. 5

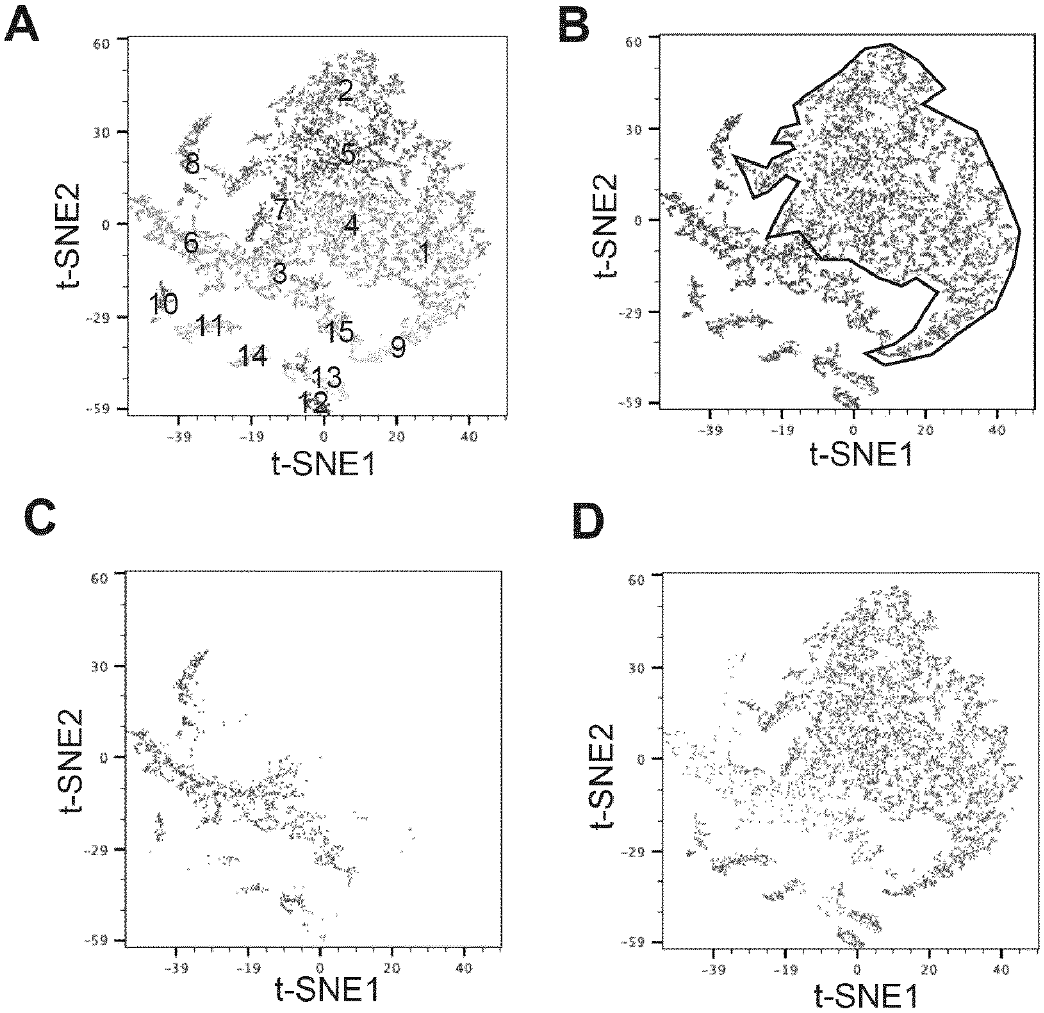
A
B
C
D
t-SNE1
t-SNE2
60
30
0
-29
-59
-39
-19
0
20
40
1
2
3
4
5
6
7
8
9
10
11
12
13
14
15

FIG. 6

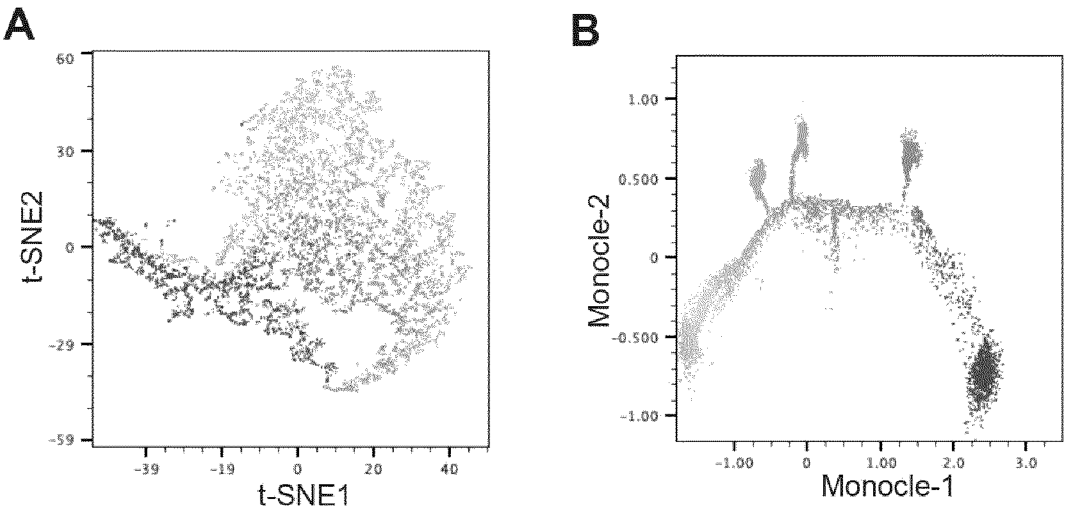
A
B
t-SNE1
t-SNE2
Monocle-1
Monocle-2
60
30
0
-29
-59
-39
-19
0
20
40
1.00
0.500
0
-0.500
-1.00
-1.00
0
1.00
2.0
3.0

FIG. 7

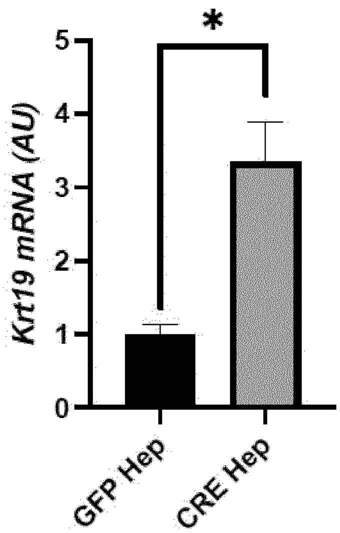
*
Krt19 mRNA (AU)
0
1
2
3
4
5
GFP Hep
CRE Hep

FIG. 8

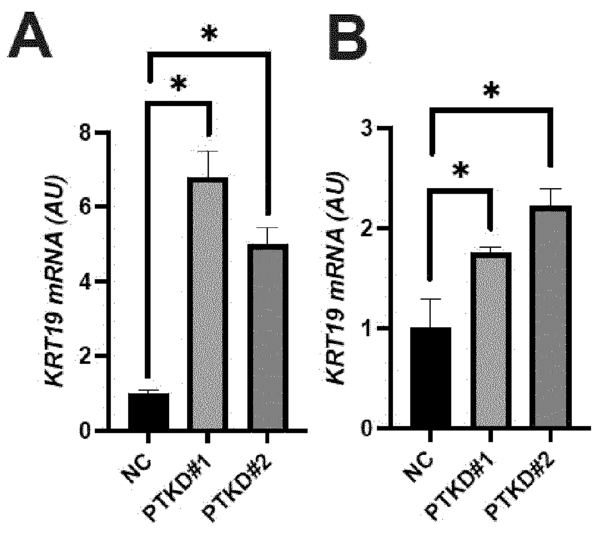
A
*
*
KRT19 mRNA (AU)
0
2
4
6
8
NC
PTKD#1
PTKD#2
B
*
*
KRT19 mRNA (AU)
0
1
2
3
NC
PTKD#1
PTKD#2

FIG. 9

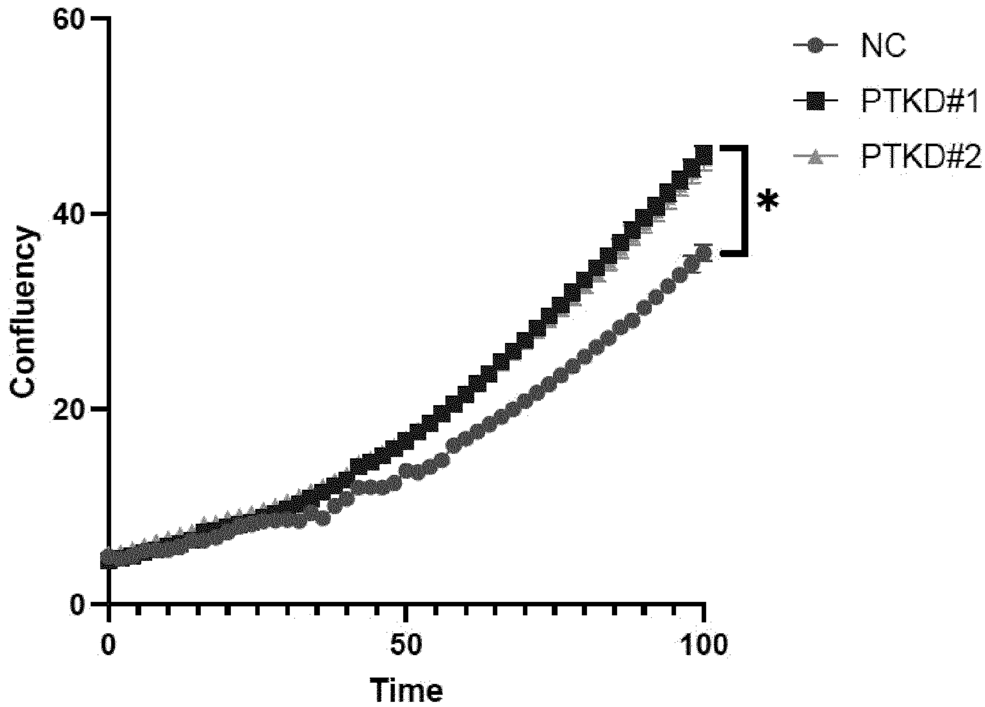

FIG. 10

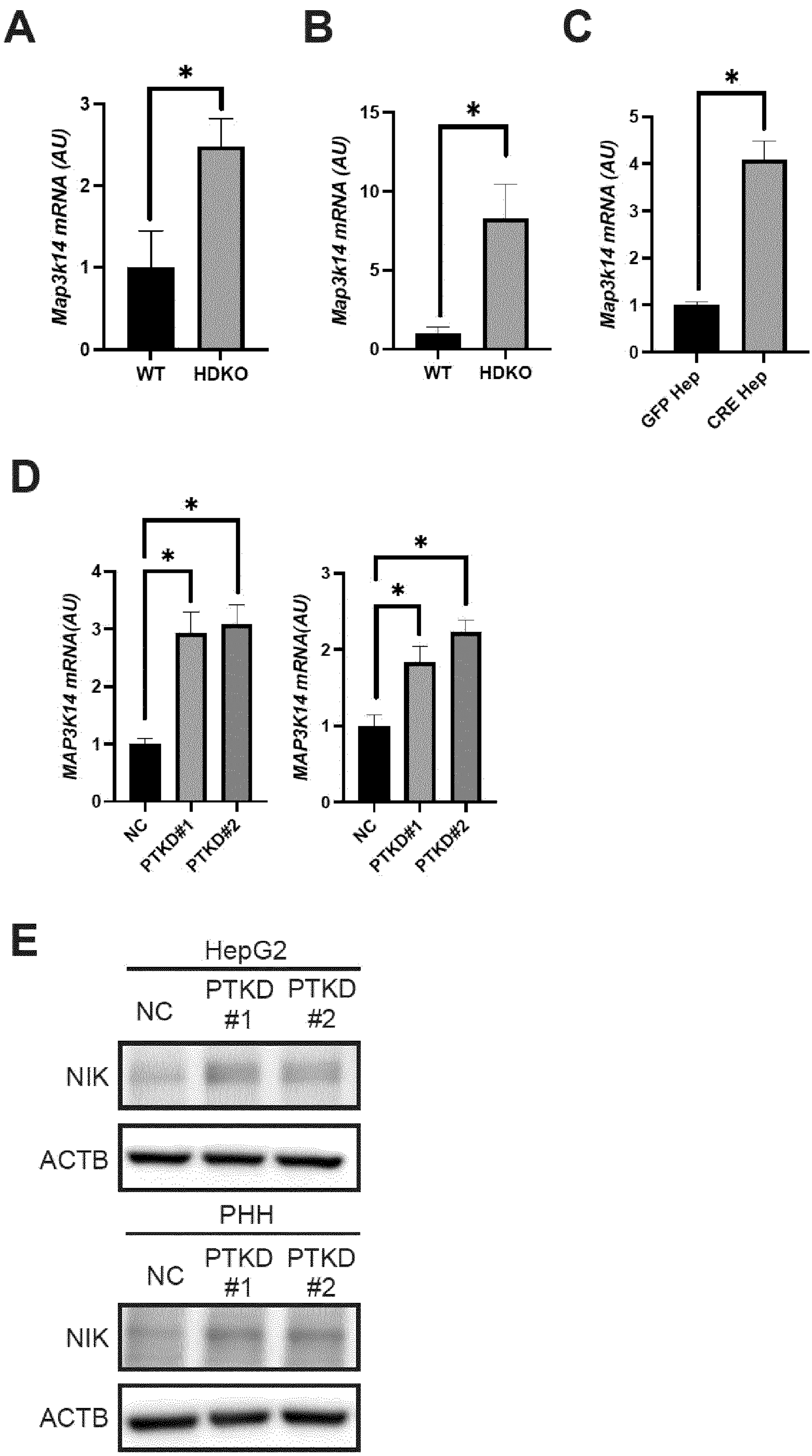
A
*
Map3k14 mRNA (AU)
3
2
1
0
WT
HDKO
B
*
Map3k14 mRNA (AU)
15
10
5
0
WT
HDKO
C
*
Map3k14 mRNA (AU)
5
4
3
2
1
0
GFP Hep
CRE Hep
D
*
*
MAP3K14 mRNA(AU)
4
3
2
1
0
NC
PTKD#1
PTKD#2
*
*
MAP3K14 mRNA(AU)
3
2
1
0
NC
PTKD#1
PTKD#2
E
HepG2
NC
PTKD #1
PTKD #2
NIK
ACTB
PHH
NC
PTKD #1
PTKD #2
NIK
ACTB

FIG. 11

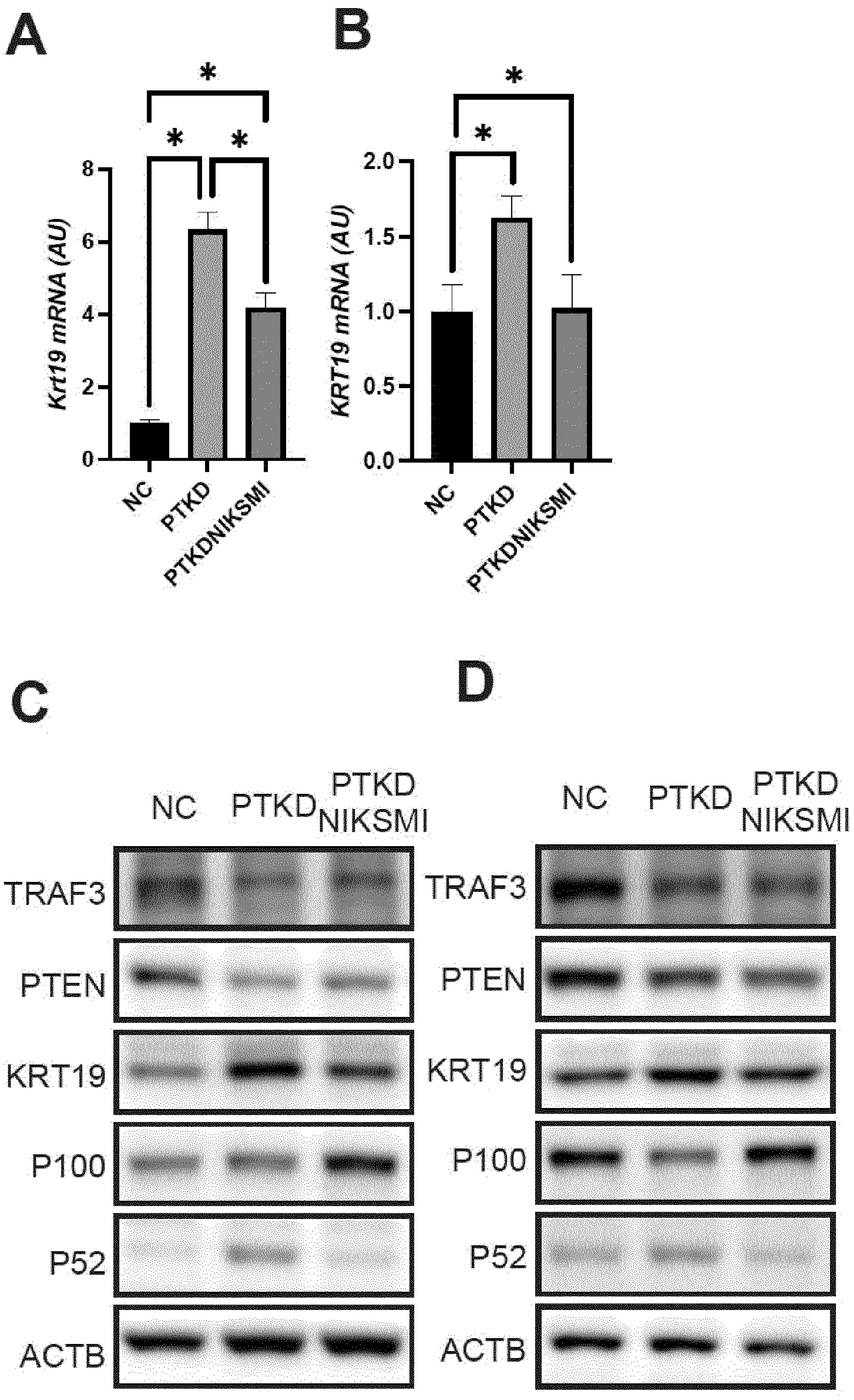
A
B
Krt19 mRNA (AU)
KRT19 mRNA (AU)
0
2
4
6
8
0.0
0.5
1.0
1.5
2.0
NC
PTKD
PTKDNIKSMI
C
D
NC
PTKD
PTKD NIKSMI
TRAF3
PTEN
KRT19
P100
P52
ACTB

FIG. 12

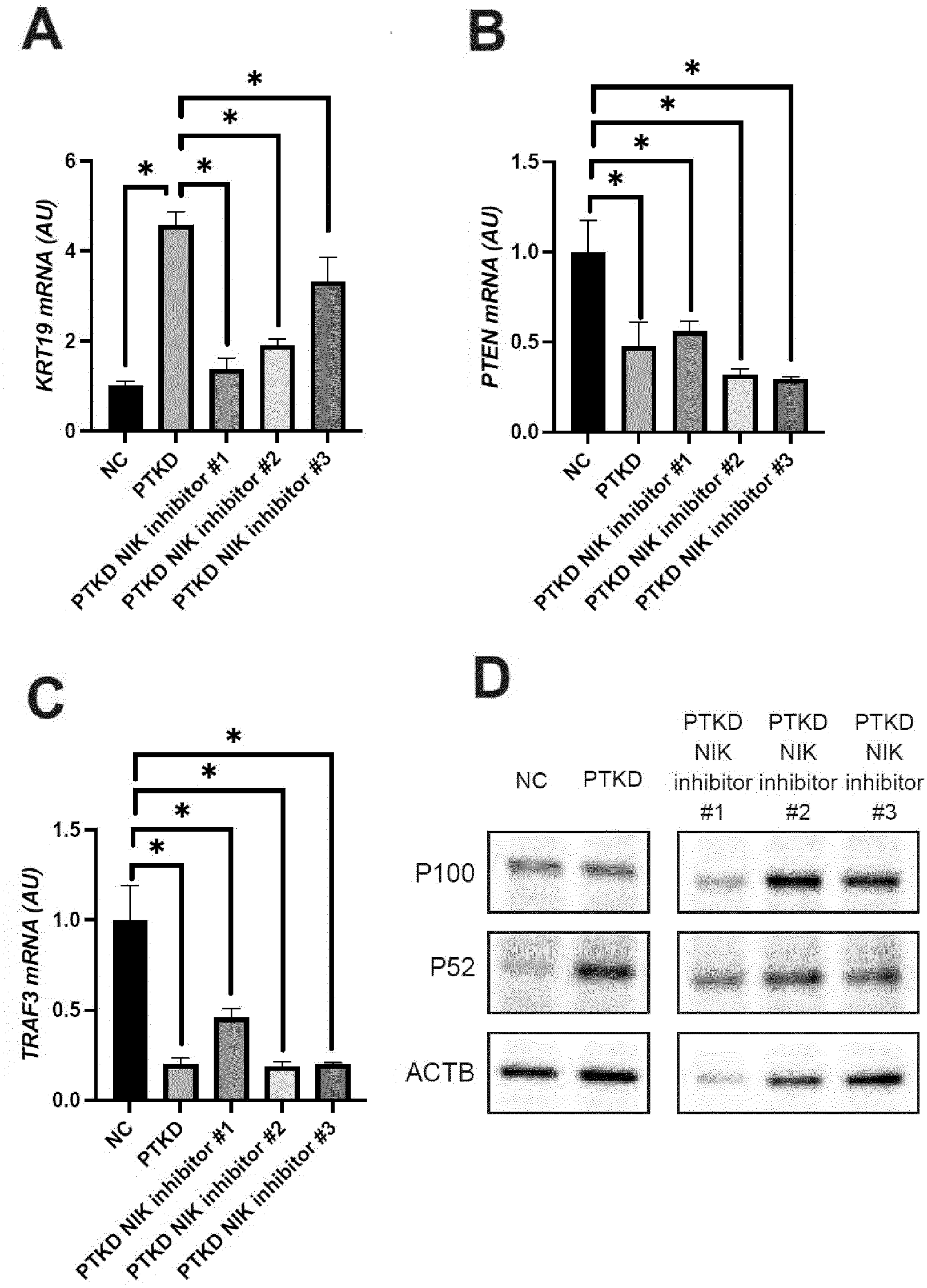
A
KRT19 mRNA (AU)
0
2
4
6
*
*
*
*
NC
PTKD
PTKD NIK inhibitor #1
PTKD NIK inhibitor #2
PTKD NIK inhibitor #3
B
PTEN mRNA (AU)
0.0
0.5
1.0
1.5
*
*
*
*
NC
PTKD
PTKD NIK inhibitor #1
PTKD NIK inhibitor #2
PTKD NIK inhibitor #3
C
TRAF3 mRNA (AU)
0.0
0.5
1.0
1.5
*
*
*
*
NC
PTKD
PTKD NIK inhibitor #1
PTKD NIK inhibitor #2
PTKD NIK inhibitor #3
D
NC
PTKD
PTKD NIK inhibitor #1
PTKD NIK inhibitor #2
PTKD NIK inhibitor #3
P100
P52
ACTB

FIG. 13

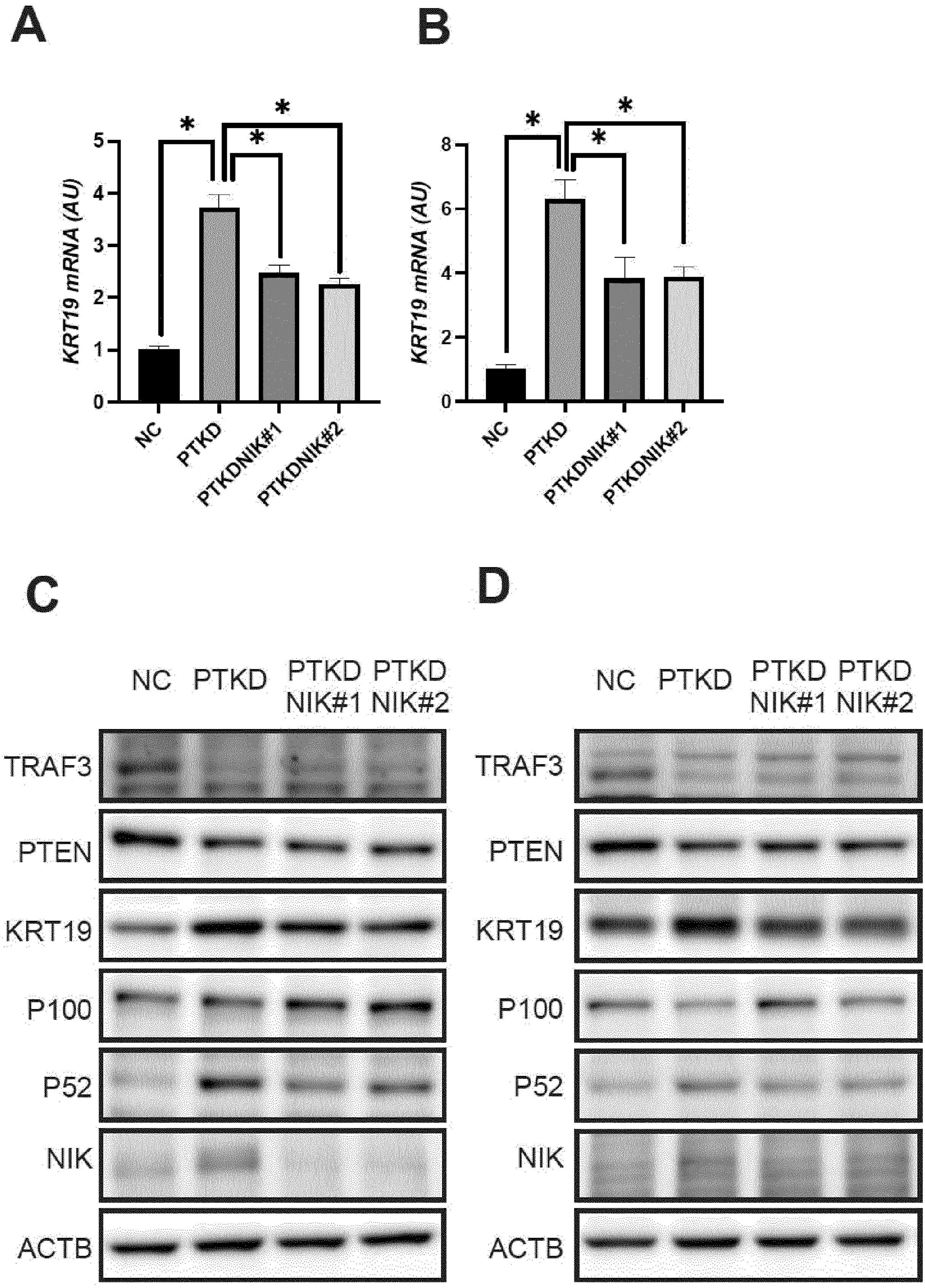
A
B
KRT19 mRNA (AU)
*
NC
PTKD
PTKDNIK#1
PTKDNIK#2
C
D
NC
PTKD
PTKD NIK#1
PTKD NIK#2
TRAF3
PTEN
KRT19
P100
P52
NIK
ACTB

FIG. 14

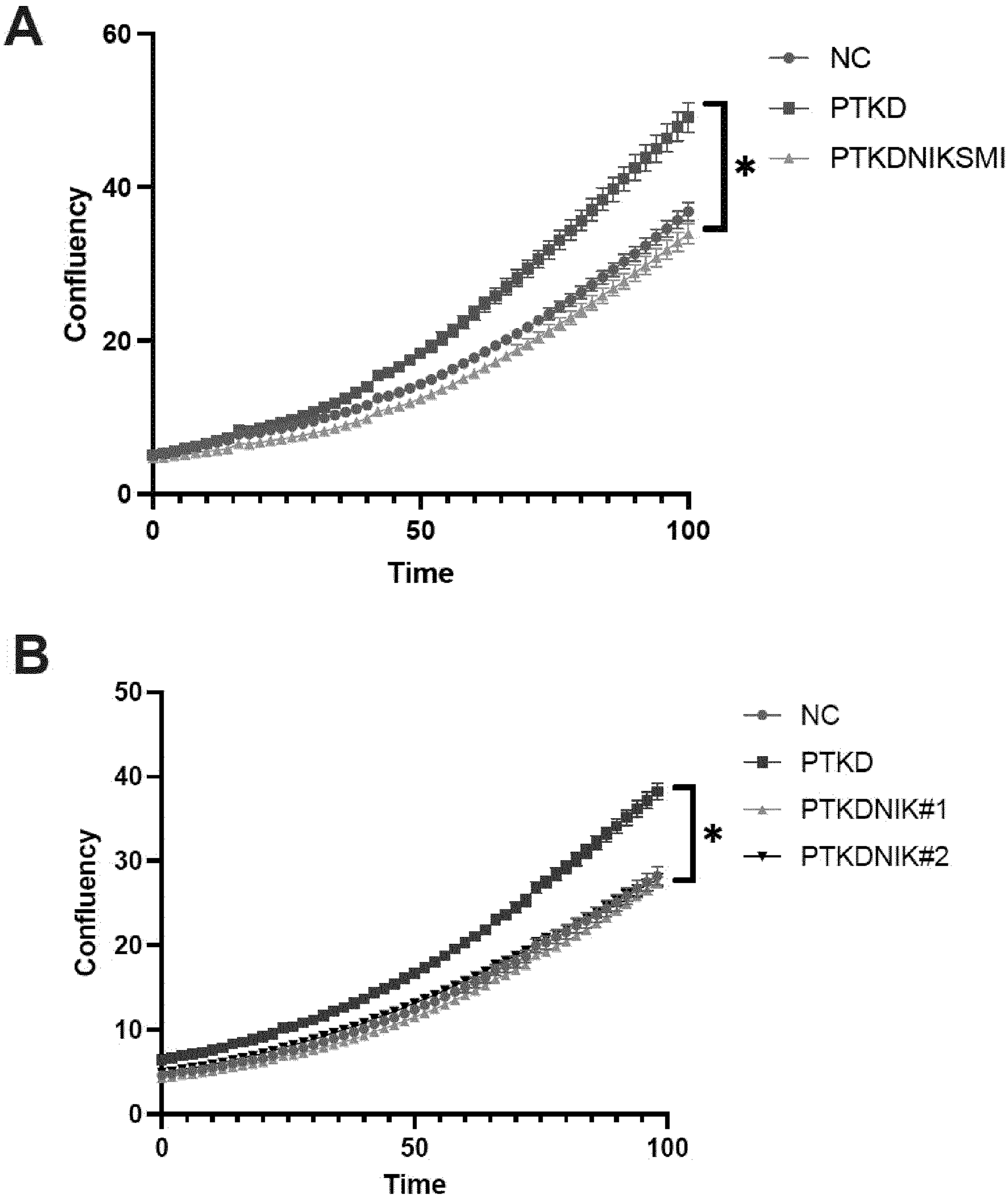
A
60
40
20
0
Confluency
0
50
100
Time
NC
PTKD
PTKDNIKSMI
*
B
50
40
30
20
10
0
Confluency
0
50
100
Time
NC
PTKD
PTKDNIK#1
PTKDNIK#2
*

FIG. 15

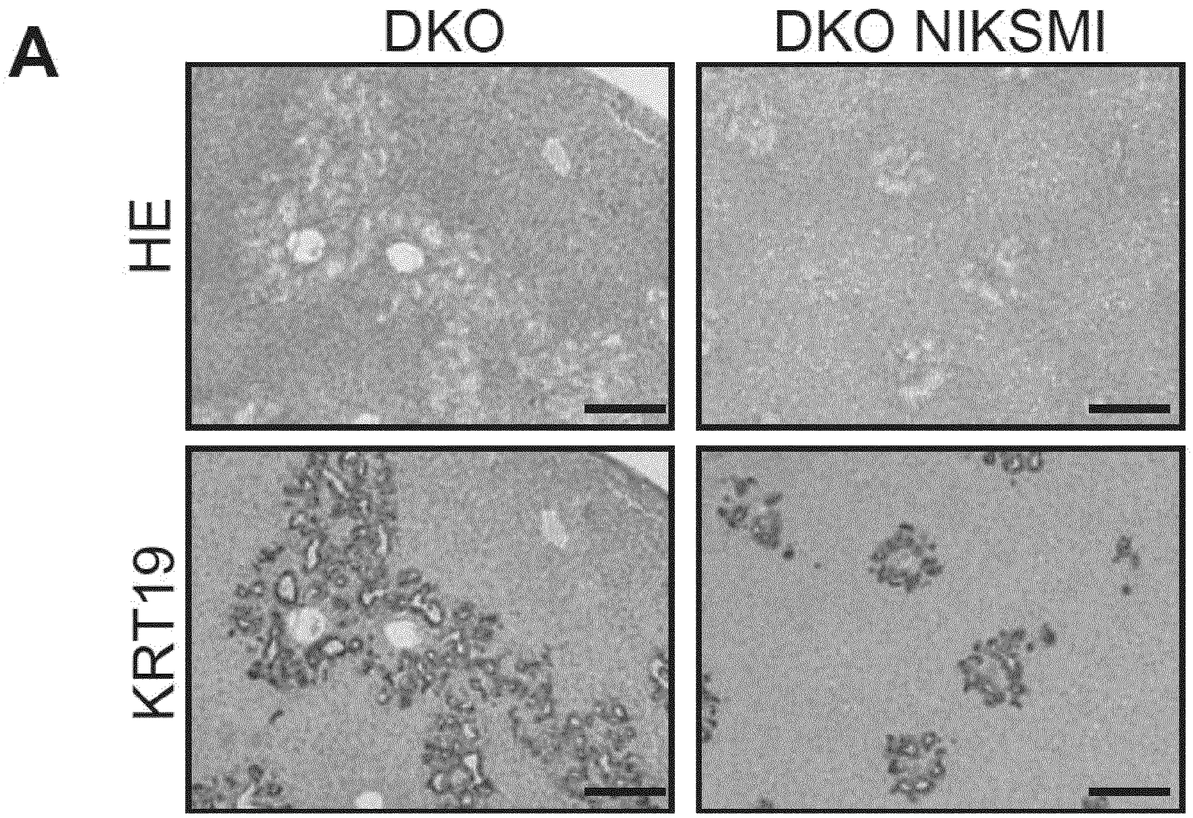
A
DKO
DKO NIKSMI
HE
KRT19

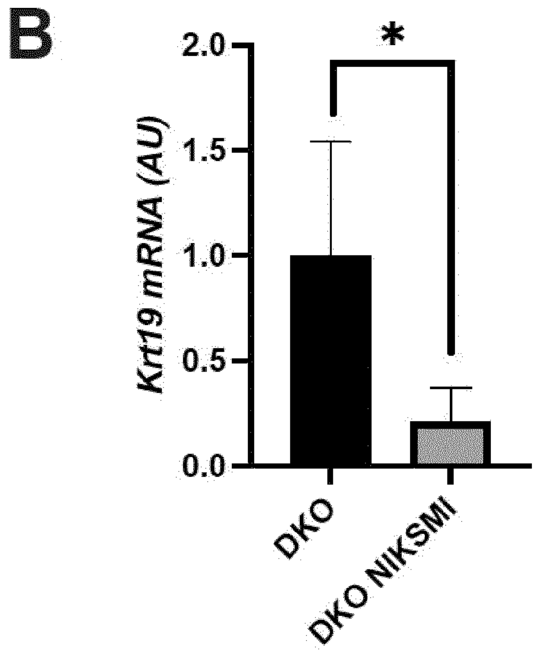
B
Krt19 mRNA (AU)
2.0
1.5
1.0
0.5
0.0
*
DKO
DKO NIKSMI

FIG. 16

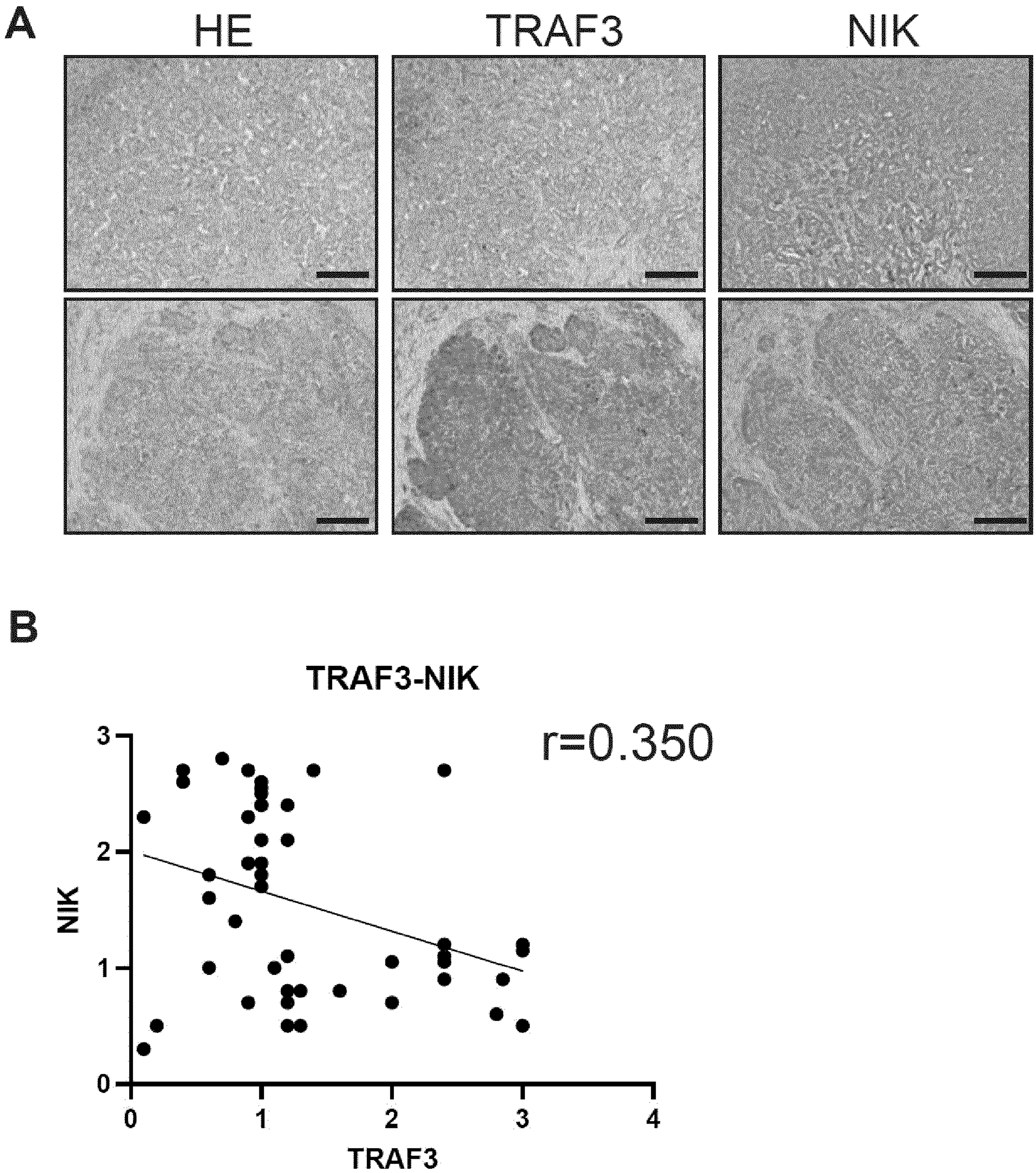

FIG. 17

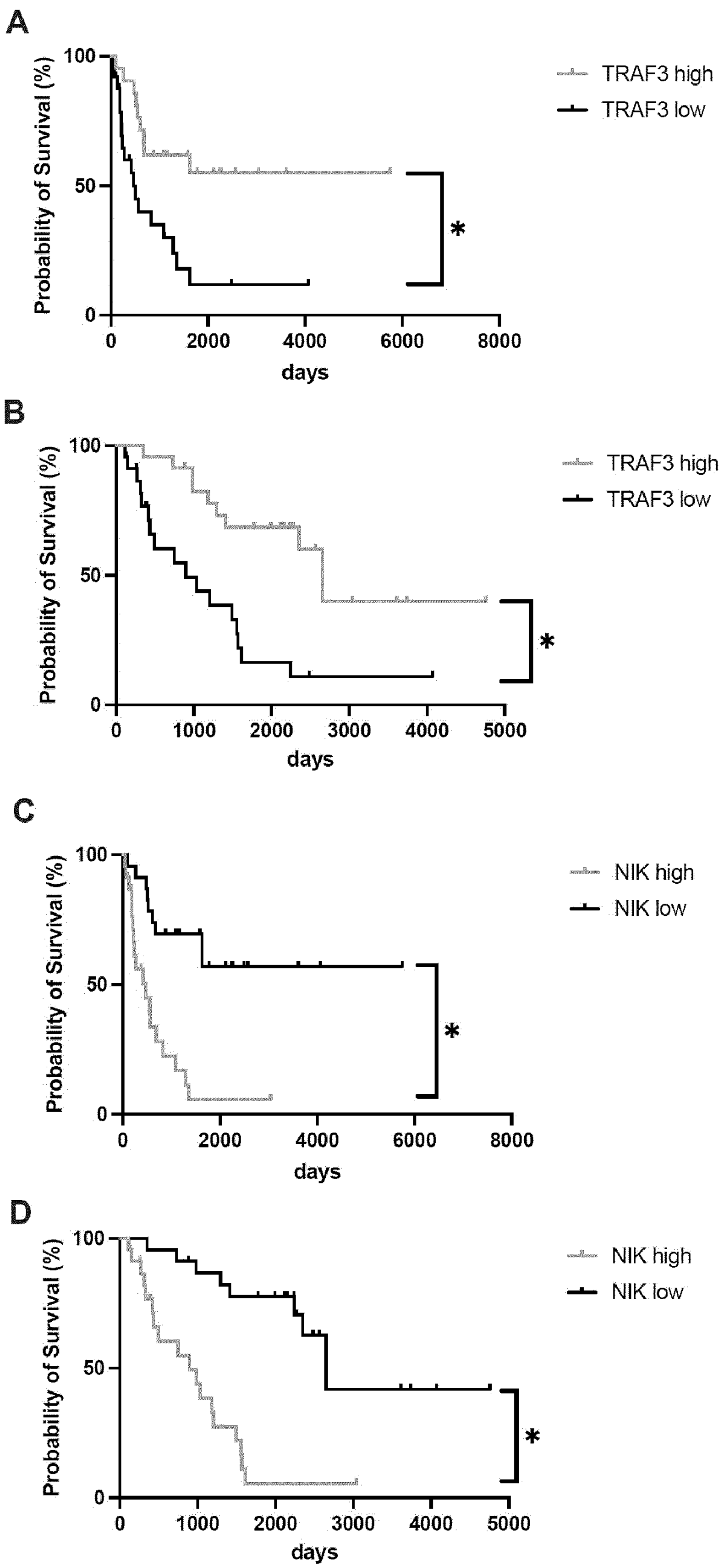
A
100
50
0
Probability of Survival (%)
0
2000
4000
6000
8000
days
TRAF3 high
TRAF3 low
*
B
100
50
0
Probability of Survival (%)
0
1000
2000
3000
4000
5000
days
TRAF3 high
TRAF3 low
*
C
100
50
0
Probability of Survival (%)
0
2000
4000
6000
8000
days
NIK high
NIK low
*
D
100
50
0
Probability of Survival (%)
0
1000
2000
3000
4000
5000
days
NIK high
NIK low
*

FIG. 18

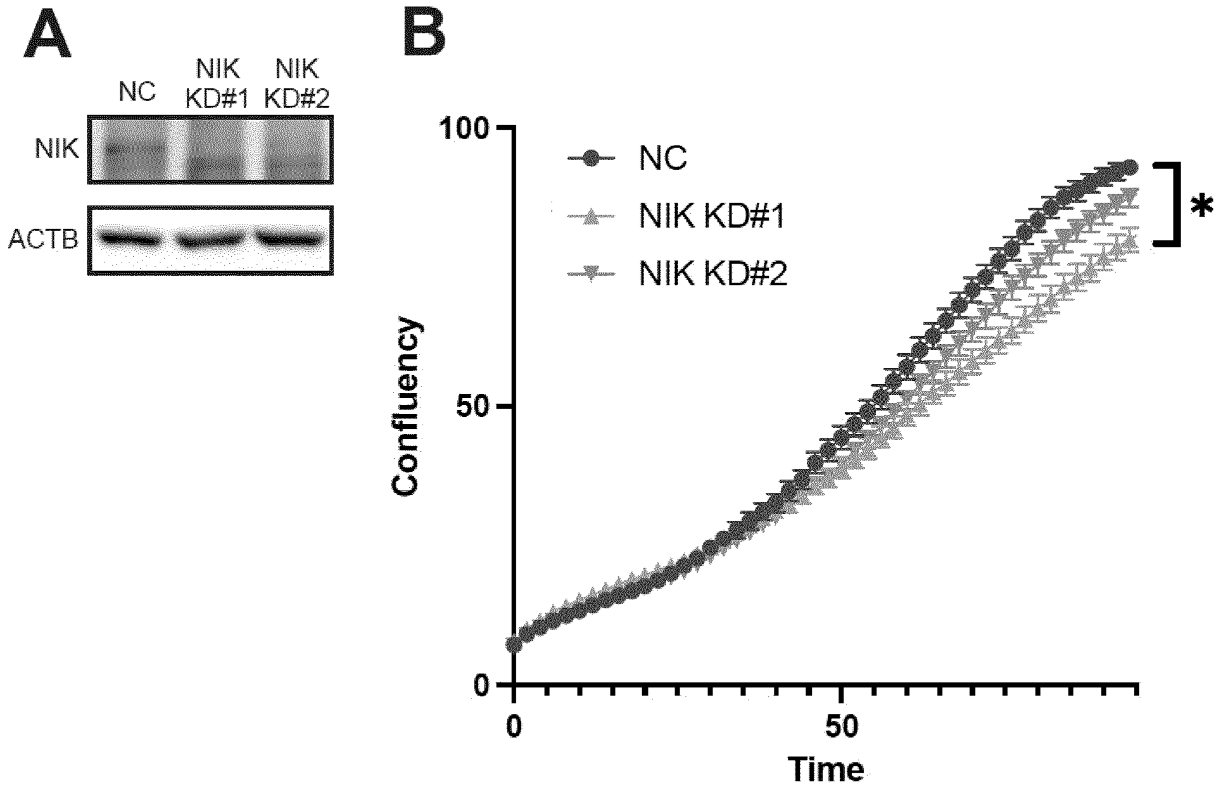
A
NC
NIK KD#1
NIK KD#2
NIK
ACTB
B
100
50
0
Confluency
0
50
Time
NC
NIK KD#1
NIK KD#2
*

FIG. 19

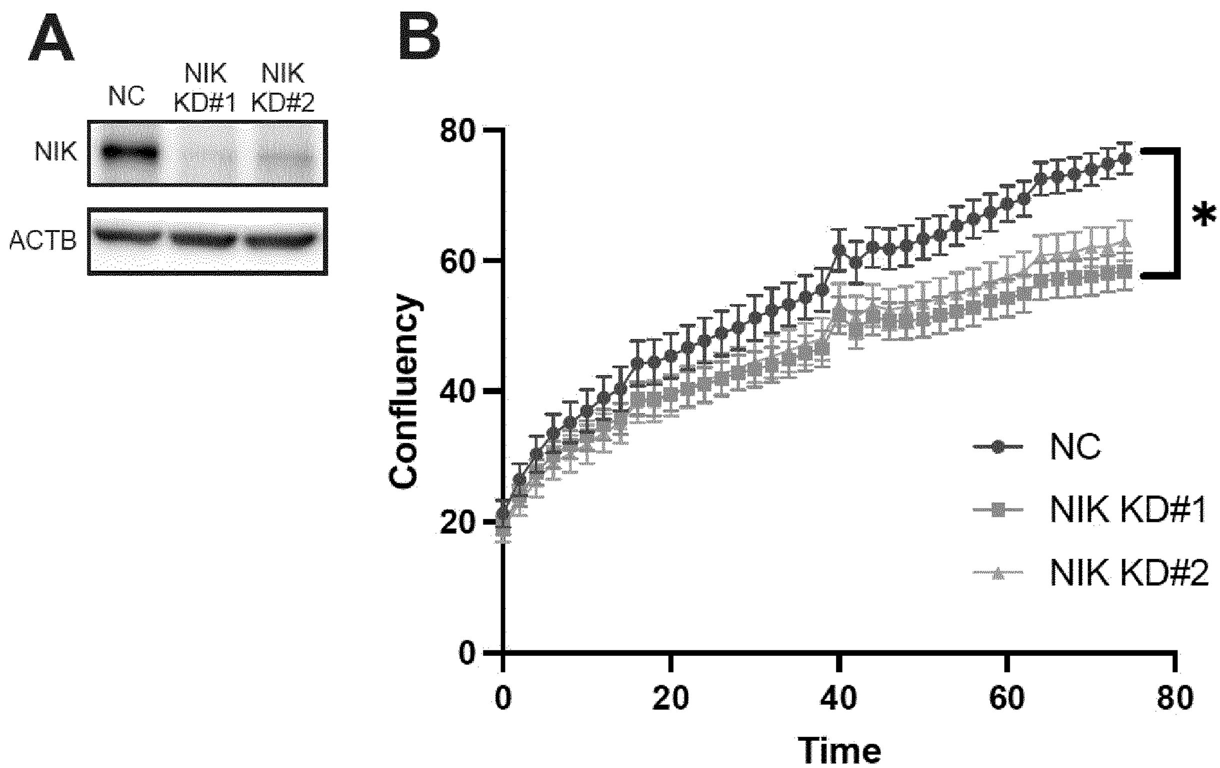
A
NC
NIK KD#1
NIK KD#2
NIK
ACTB
B
80
60
40
20
0
Confluency
0
20
40
60
80
Time
NC
NIK KD#1
NIK KD#2
*

FIG. 20

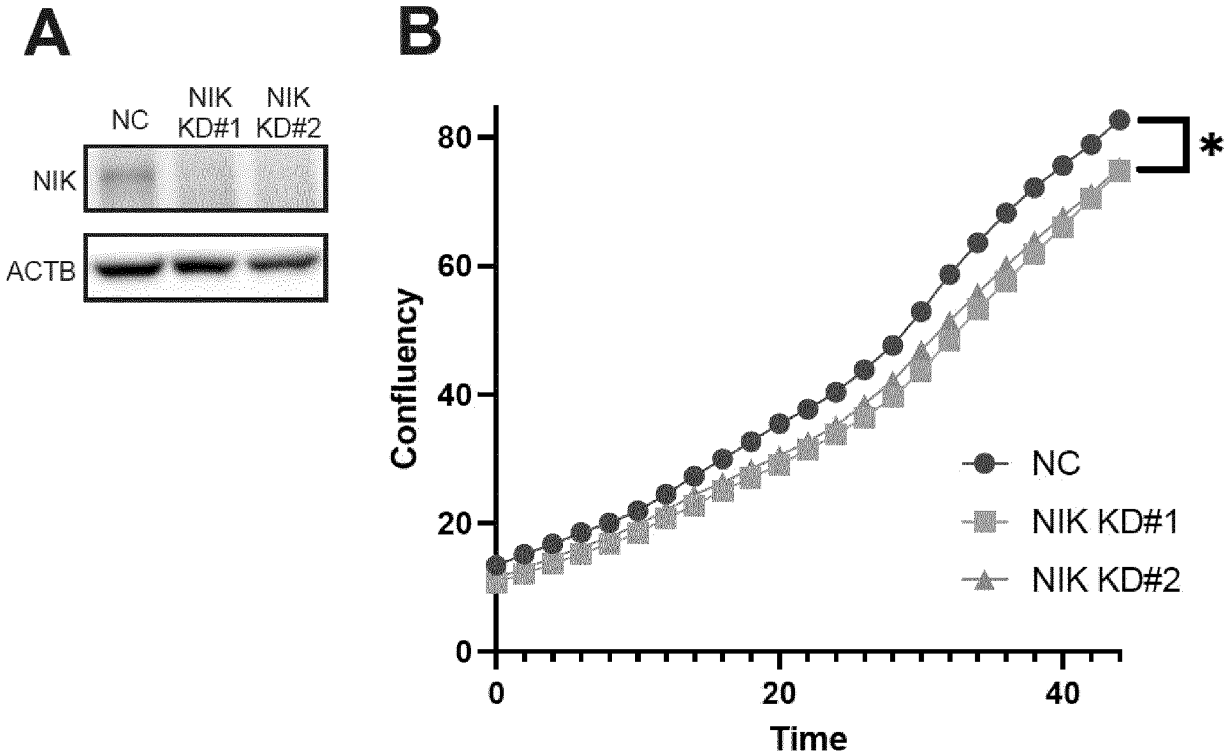
A
NC
NIK KD#1
NIK KD#2
NIK
ACTB
B
Confluency
80
60
40
20
0
0
20
40
Time
*
NC
NIK KD#1
NIK KD#2

FIG. 21

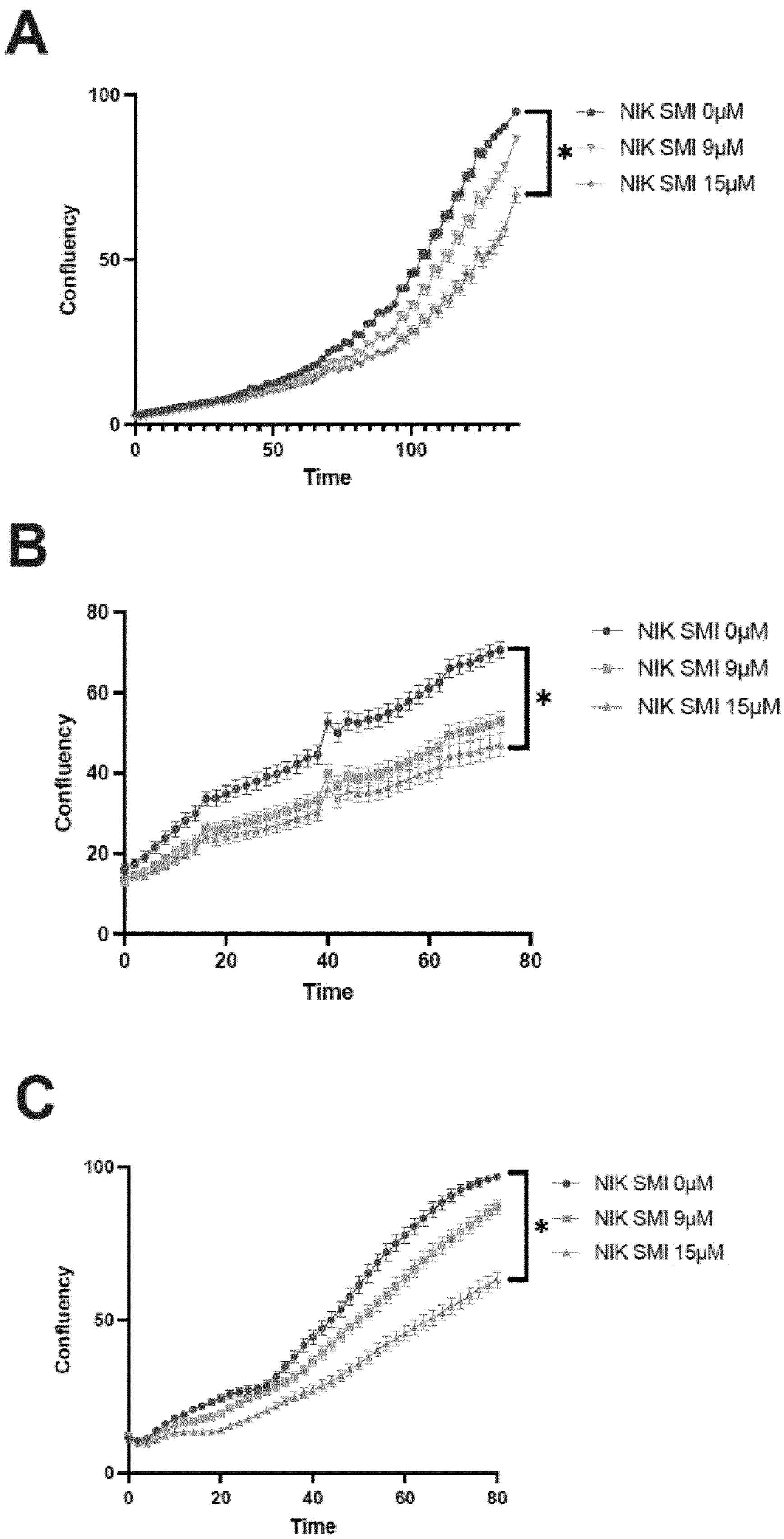
A
100
50
0
0
50
100
Confluency
Time
*
NIK SMI 0μM
NIK SMI 9μM
NIK SMI 15μM
B
80
60
40
20
0
0
20
40
60
80
Confluency
Time
*
NIK SMI 0μM
NIK SMI 9μM
NIK SMI 15μM
C
100
50
0
0
20
40
60
80
Confluency
Time
*
NIK SMI 0μM
NIK SMI 9μM
NIK SMI 15μM

FIG. 22

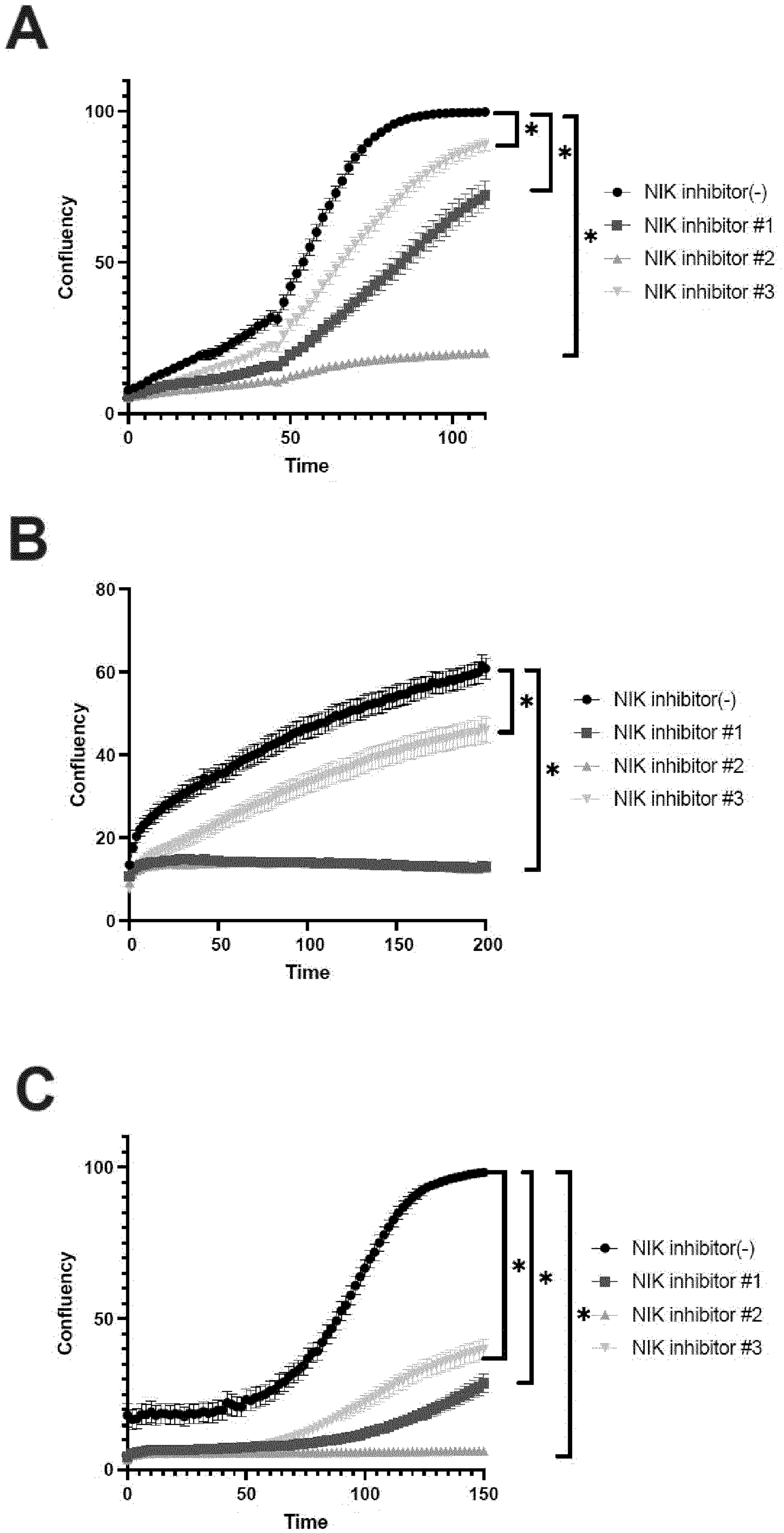
A
100
50
0
Confluency
0
50
100
Time
*
*
*
NIK inhibitor(-)
NIK inhibitor #1
NIK inhibitor #2
NIK inhibitor #3
B
80
60
40
20
0
Confluency
0
50
100
150
200
Time
*
*
NIK inhibitor(-)
NIK inhibitor #1
NIK inhibitor #2
NIK inhibitor #3
C
100
50
0
Confluency
0
50
100
150
Time
*
*
*
NIK inhibitor(-)
NIK inhibitor #1
NIK inhibitor #2
NIK inhibitor #3

FIG. 23

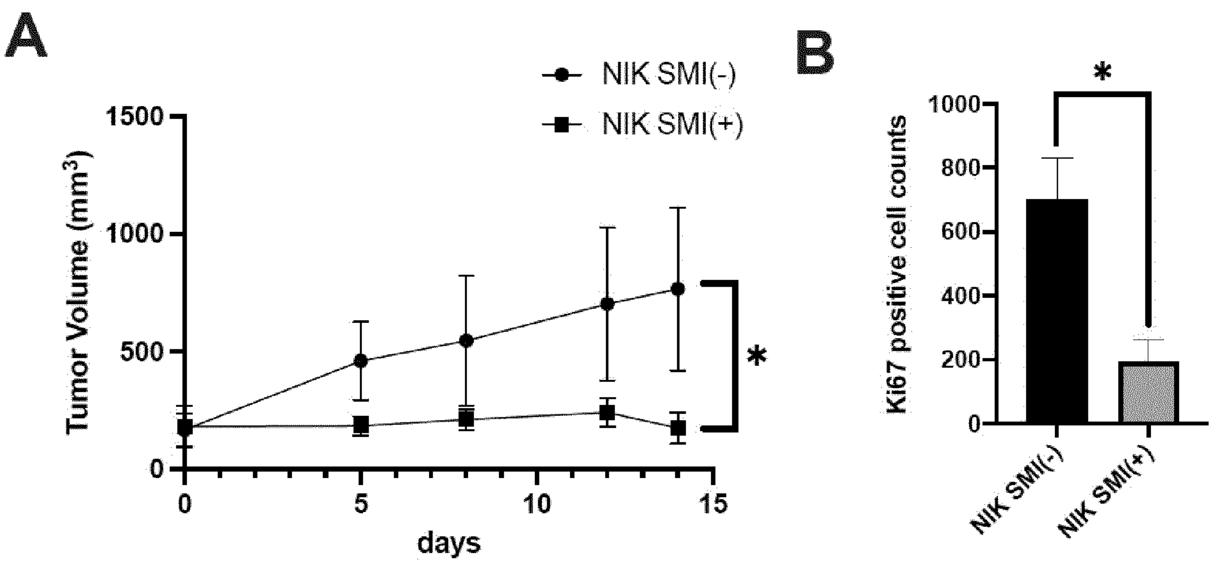
A
NIK SMI(-)
NIK SMI(+)
Tumor Volume (mm3)
1500
1000
500
0
0
5
10
15
days
*
B
Ki67 positive cell counts
1000
800
600
400
200
0
NIK SMI(-)
NIK SMI(+)
*

FIG. 24

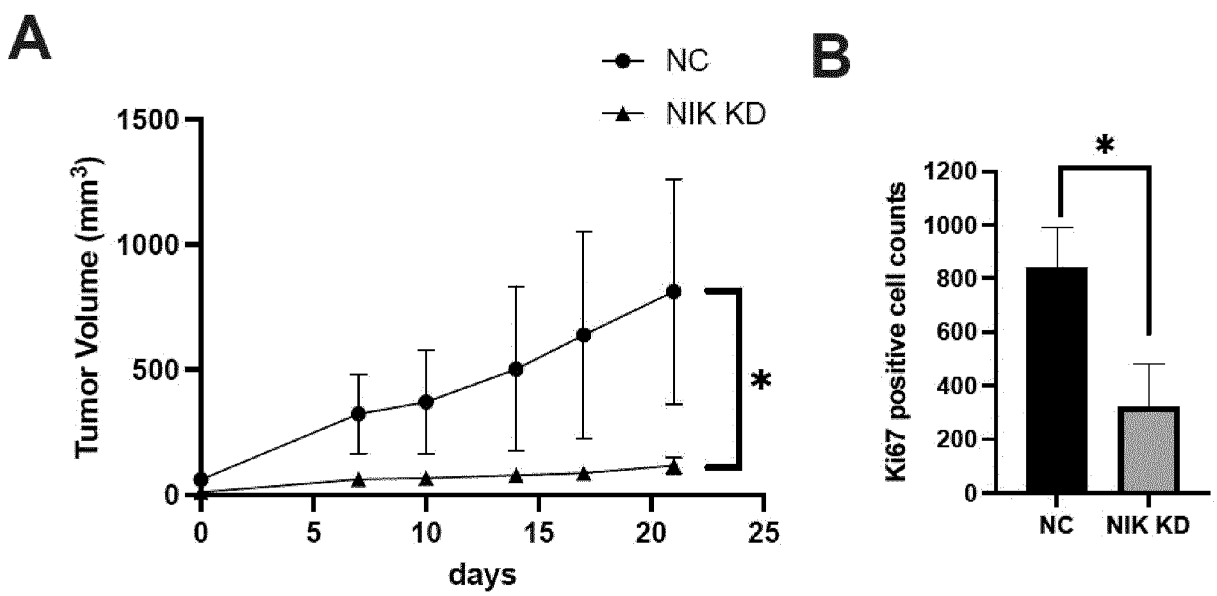
A
NC
NIK KD
Tumor Volume (mm3)
1500
1000
500
0
0
5
10
15
20
25
days
*
B
Ki67 positive cell counts
1200
1000
800
600
400
200
0
NC
NIK KD
*

**INTERNATIONAL SEARCH REPORT**

International application No.
**PCT/JP2022/029655**

**A. CLASSIFICATION OF SUBJECT MATTER**

***A61K 45/00***(2006.01)i; ***A61K 31/4439***(2006.01)i; ***A61K 31/7088***(2006.01)i; ***A61K 48/00***(2006.01)i; ***A61P 1/16***(2006.01)i; ***A61P 35/00***(2006.01)i; ***A61P 43/00***(2006.01)i; ***C12Q 1/06***(2006.01)i; ***C12Q 1/48***(2006.01)i; ***G01N 33/15***(2006.01)i; ***G01N 33/50***(2006.01)i

FI: A61K45/00 ZNA; A61P1/16; A61P35/00; A61P43/00 111; A61K48/00; A61K31/7088; A61K31/4439; C12Q1/06; C12Q1/48 Z; G01N33/15 Z; G01N33/50 Z

According to International Patent Classification (IPC) or to both national classification and IPC

**B. FIELDS SEARCHED**

Minimum documentation searched (classification system followed by classification symbols)

A61K45/00; A61K31/4439; A61K31/7088; A61K48/00; A61P1/16; A61P35/00; A61P43/00; C12Q1/06; C12Q1/48; G01N33/15; G01N33/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | LU, W.-X. Long non-coding RNA MEG3 represses cholangiocarcinoma by regulating miR-361-5p/TRAF3 axis. European Review for Medical and Pharmacological Sciences. 2019, vol. 23, no. 17, pp. 7356-7368<br>abstract | 1-8, 12, 13 |
| Y | | 1-15 |
| X | ZHANG, S. et al. MicroRNA-520e suppresses growth of hepatoma cells by targeting the NF-κB-inducing kinase (NIK). Oncogene. November 2011, vol. 31, pp. 3607-3620, https://doi.org/10.1038/onc.2011.523<br>abstract, NIK contributes to miR-520e-suppressed cell growth, Expression of miR-520e is frequently reduced in hepatomacell lines and HCC tissues, fig. 6d | 1-8, 11-13, 15 |
| Y | | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2022** | **13 September 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/029655**

**C. DOCUMENTS CONSIDERED TO BE RELEVANT**

| Category* | Citation of document, with indication, where appropriate, of the relevant passages | Relevant to claim No. |
|---|---|---|
| Y | LI, Bo-an. A novel tumor suppressor miRNA miR-520e contributes to suppression of hepatoma. Acta Pharmacologica Sinica. 2012, 33(1), pp. 3, 4, https://doi.org/10.1038/aps.2011.198<br>p. 3, right column, lines 13-3 from the bottom | 1-15 |
| Y | SHIODE, Yuto et al. Inactivation of traf3 promotes intrahepatic cholangiocarcinoma development via hepatocyte trans-differentiation. Cancer Research. 01 July 2021, vol. 81, no. 13 suppl, abstract number: 2927, https://doi.org/10.1158/1538-7445.AM2021-2927<br>abstract | 1-15 |
| Y | JP 2016-531127 A (F. HOFFMANN-LA ROCHE AG) 06 October 2016 (2016-10-06)<br>claims, examples | 8-10 |
| P, X | SHIODE, Yuto et al. TNF receptor-related factor 3 inactivation promotes the development of intrahepatic cholangiocarcinoma through NF-κB-inducing kinase-mediated hepatocyte transdifferentiation. Hepatology. 07 January 2022, pp. 1-17, https://doi.org/10.1002/hep.32317<br>abstract | 1-15 |

Form PCT/ISA/210 (second sheet) (January 2015)

**INTERNATIONAL SEARCH REPORT**

International application No.
**PCT/JP2022/029655**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

The “form of an Annex CST.25 text file” is replaced with the “form of ST.26.”.

Form PCT/ISA/210 (continuation of first sheet) (January 2015)

**INTERNATIONAL SEARCH REPORT**
**Information on patent family members**

International application No.
**PCT/JP2022/029655**

| Patent document cited in search report | Publication date (day/month/year) | Patent family member(s) | Publication date (day/month/year) |
|---|---|---|---|
| JP 2016-531127 A | 06 October 2016 | WO 2015/025026 A1<br>claims, examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015025026 A1
- WO 2018037059 A1
- WO 2017125530 A1


### Non-patent literature cited in the description


- **RIZVI S et al.** *Nat Rev Clin Oncol.,* 2018, vol. 15 (2), 95-111
- **BANALES JM et al.** *Nat Rev Gastroenterol Hepatol.,* 2020, vol. 17 (9), 557-88
- **FARSHIDFAR F et al.** *Cell Rep.,* 2017, vol. 19 (13), 2878-80
- **KODAMA T et al.** *Gastroenterology,* 2016, vol. 151 (2), 324-37 e12
- **KODAMA T et al.** *Proc Natl Acad Sci U S A.,* 2016, vol. 113 (24), E3384-93
- **SHIODE YUTO et al.** *The 57th Annual Meeting of the Japan Society of Hepatology,* 2021, WS9-10
- **BRIGHTBILL HD et al.** *Nat Commun,* 2018, vol. 9 (1), 179
- **NISHIKAWA et al.** *the 109th Annual Meeting of the Japanese Society of Pathology*
- **SCHULER M et al.** *Genesis,* 2004, vol. 39 (3), 167-172
- **KODAMA T et al.** *Proc Natl Acad Sci USA.,* 2018, vol. 115 (44), E10417-E10426
- **KODAMA T et al.** *Gastroenterology,* 2016, vol. 151 (2), 324-37e12
- **HASEGAWA M et al.** *Biochem Biophys Res Commun.,* 2011, vol. 405 (3), 405-410
- **MURAI K et al.** *Sci Rep.,* 2020, vol. 10 (1), 941
- **BROUTIER L et al.** *Nat Protoc.,* 2016, vol. 11 (9), 1724-1743
- **TANAKA S et al.** *Hepatology,* 2016, vol. 64 (6), 1994-2014
- **HORIE Y et al.** *J Clin Invest.,* 2004, vol. 113 (12), 1774-1783
- **MANN KM et al.** *Nat Biotechnol.,* 2016, vol. 34 (9), 962-972
- **SORIANO P.** *Nat Genet.,* 1999, vol. 21 (1), 70-71
- **YANGER K et al.** *Cell Stem Cell,* 2014, vol. 15 (3), 340-349
- **SEEHAWER M et al.** *Nature,* 2018, vol. 562 (7725), 69-75
- **PARVATIYAR K et al.** *Nat Commun.,* 2018, vol. 9 (1), 2770